(19) Europäisches Patentamt
European Patent Office
Office européen des brevets

(11) EP 1 060 175 B1

(12) **EUROPÄISCHE PATENTSCHRIFT**

(45) Veröffentlichungstag und Bekanntmachung des Hinweises auf die Patenterteilung:
**17.11.2004 Patentblatt 2004/47**

(21) Anmeldenummer: **99910192.6**

(22) Anmeldetag: **04.02.1999**

(51) Int Cl.[7]: **C07D 401/12**, C07D 295/192, A61K 31/44

(86) Internationale Anmeldenummer:
**PCT/EP1999/000726**

(87) Internationale Veröffentlichungsnummer:
**WO 1999/040083 (12.08.1999 Gazette 1999/32)**

(54) **TRYPTASE-INHIBITOREN**
TRYPTASE INHIBITORS
INHIBITEURS DE LA TRYPTASE

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **06.02.1998 DE 19804761**
**06.11.1998 DE 19851299**

(43) Veröffentlichungstag der Anmeldung:
**20.12.2000 Patentblatt 2000/51**

(73) Patentinhaber:
- **Max-Planck-Gesellschaft zur Förderung der Wissenschaften e.V. 80539 München (DE)**
- **ALTANA Pharma AG 78467 Konstanz (DE)**

(72) Erfinder:
- **BODE, Wolfram D-82131 Gauting (DE)**
- **MORODER, Luis D-82152 Martinsried (DE)**
- **PEREIRA, Pedro, Jose, Barbosa D-82152 Krailling (DE)**
- **BERGNER, Andreas D-82061 Neuried (DE)**
- **HUBER, Robert D-82110 Germering (DE)**
- **SOMMERHOFF, Christian D-81927 München (DE)**
- **SCHASCHKE, Norbert D-81373 München (DE)**
- **BÄR, Thomas D-78479 Reichenau (DE)**
- **MARTIN, Thomas D-78462 Konstanz (DE)**
- **STADLWIESER, Josef D-78465 Konstanz (DE)**
- **ULRICH, Wolf-Rüdiger D-78462 Konstanz (DE)**
- **DOMINIK, Andreas D-78333 Stockach (DE)**
- **THIBAUT, Ulrich D-78464 Konstanz (DE)**
- **BUNDSCHUH, Daniela D-78462 Konstanz (DE)**
- **BEUME, Rolf D-78465 Konstanz (DE)**
- **GOEBEL, Karl-Josef D-78333 Stockach (DE)**

(74) Vertreter: **Huber, Bernhard, Dipl.-Chem. et al Weickmann & Weickmann Patentanwälte Postfach 86 08 20 81635 München (DE)**

(56) Entgegenhaltungen:
**WO-A-95/32945** **WO-A-96/09297**
**WO-A-98/04537**

- **RICE,K.D. ET AL.: "Inhibitors of Tryptase for the treatment of Mast Cell-Mediated Diseases" CURR.PHARM.DES., Bd. 4, Nr. 5, Mai 1998 (1998-05), Seiten 381-396, XP002108322**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 060 175 B1

- CAUGHEY G H ET AL: "BIS(5-AMIDINO-2-BENZIMIDAZOLYL)METHANE AND RELATED AMIDINES. ARE POTENT, REVERSIBLE INHIBITORS OF MAST CELL TRYPTASES" JOURNAL OF PHARMACOLOGY AND EXPERIMENTAL THERAPEUTICS, Bd. 264, Nr. 2, 1. Januar 1993 (1993-01-01), Seiten 676-682, XP002064911
- STÜRZEBECHER, J. ET AL.: "Inhibition of Human Mast Cell Tryptase by Benzamidine Derivatives" BIOL.CHEM.HOPPE-SEYLER, Bd. 373, Oktober 1992 (1992-10), Seiten 1025-10303, XP002108323 BERLIN
- GOODSON,J.A. ET AL.: "The Chemotherapy of Amoebiasis. III. Variants of Bis(diamylamino)decane" BR.J.PHARMACOL., Bd. 3, 1948, Seiten 62-71, XP002108875 LONDON

## Beschreibung

### Anwendung der Erfindung

Die Erfindung betrifft neue Inhibitoren von humaner Tryptase, die in der pharmazeutischen Industrie zur Herstellung von Medikamenten verwendet werden.

### Bekannter technischer Hintergrund

Humane Tryptase ist eine Serinproteinase, die in humanen Mastzellen das überwiegend vorliegende Protein darstellt. Tryptase umfaßt vier eng verwandte Enzyme (α, I, II/β, III; mit 90 bis 98 % Sequenzidentität) (vgl. Miller et al., J.Clin.Invest. 84 (1989) 1188-1195; Miller et al., J.Clin.Invest. 86 (1990) 864-870; Vanderslice et al., Proc.Natl. Acad.Sci., USA 87 (1990) 3811-3815). Mit Ausnahme der α-Tryptase (Schwartz et al., J.Clin.Invest. 96 (1995) 2702-2710; Sakai et al., J.Clin.Invest. 97 (1996) 988-995) werden die Enzyme intrazellulär aktiviert und in katalytisch aktiver Form in Sekretgranulen gelagert.

Tryptase weist im Vergleich zu anderen bekannten Serinproteinasen, wie z.B. Trypsin oder Chymotrypsin einige besondere Eigenschaften auf (Schwartz et al., Methods Enzymol. 244, (1994), 88-100; G.H. Caughey, "Mast cell proteases in immunology and biology." Marcel Dekker, Inc., New York, 1995). Tryptase aus humanem Gewebe weist eine nicht kovalent verknüpfte tetramere Struktur auf, die durch Heparin oder andere Proteoglycane stabilisiert sein muß, um proteolytisch aktiv zu sein.

In den internationalen Anmeldungen WO95/32945, WO96/09297 und WO98/04537 werden niedermolekulare Verbindungen als Inhibitoren der Tryptase beschrieben.

### Beschreibung der Erfindung

Es wurde nun gefunden, daß die nachfolgend näher beschriebenen Verbindungen der Formel I überraschende und besonders vorteilhafte Eigenschaften besitzen.

Gegenstand der Erfindung sind Verbindungen der Formel 1

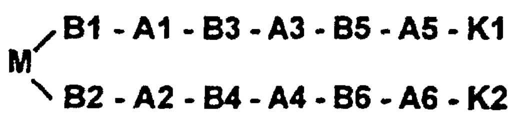

(I)

worin

A1 und A2 gleich oder verschieden sind und -O- (Sauerstoff) oder -NH-C(O)- bedeuten,

A3 und A4 gleich oder verschieden sind und -C(O)-NH- bedeuten oder ausgewählt sind aus der Gruppe

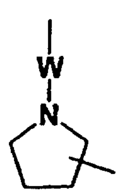 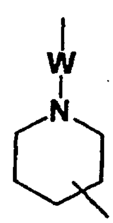 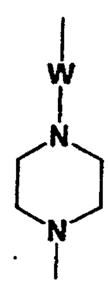

wobei W die Gruppe -C(O)- oder eine Bindung bedeutet,

A5 und A6 gleich oder verschieden sind und -C(O)-, -C(O)-NH-, -NH-C(O)- oder eine Bindung bedeuten,

M ausgewählt ist aus einer der nachfolgenden Gruppen

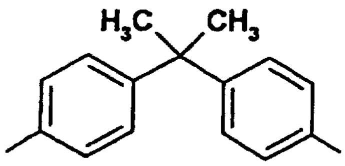 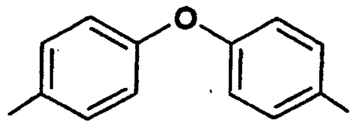 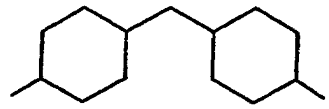

| | |
|---|---|
| K1 | -B7-(C(O))$_m$-B9-X1 oder -B7-(C(O))$_m$-B9-Z1-B11-X1 bedeutet, |
| K2 | -B8-(C(O))$_p$-B10-X2 oder -B8-(C(O))$_p$-B10-Z2-B12-X2 bedeutet, |
| B1, B2, B3, B4, B5 und B6 | gleich oder verschieden sind und eine Bindung oder -$CH_2$- (Methylen) bedeuten, |
| B7, B8, B9, B10, B11 und B12 | gleich oder verschieden sind und eine Bindung oder 1-2C-Alkylen bedeuten, |
| m | 0 oder 1 bedeutet, |
| p | 0 oder 1 bedeutet, |
| X1 und X2 | gleich oder verschieden sind und Amino, Amidino oder Guanidino bedeuten, |
| Z1 und Z2 | gleich oder verschieden sind und 1,4-Phenylen, 1,3-Phenylen, 1,4-Cyclohexylen oder 1,4-Piperazinylen bedeuten, |

und worin auf direktem Weg zwischen den terminalen Stickstoffatomen 24 bis 40 Bindungen vorhanden sein müssen, die Salze dieser Verbindungen, wobei alle diejenigen Verbindungen ausgeschlossen sind bei denen eine oder mehrere der Variablen B1, B2, B3, B4, B5, B6, B7, B8, B9, B10, B11 oder B12 die Bedeutung einer Bindung annehmen und es dadurch zur direkten Verknüpfung zweier Heteroatome oder zweier Carbonylgruppen kommen würde.

Bevorzugte Verbindungen sind solche, worin

| | |
|---|---|
| A1 und A2 | gleich oder verschieden sind und -O- (Sauerstoff) oder -NH-C(O)- bedeuten, |
| A3 und A4 | gleich oder verschieden sind und -C(O)-NH- bedeuten oder ausgewählt sind aus der Gruppe |

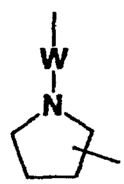 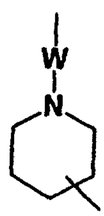 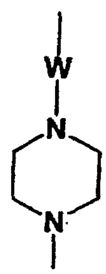

wobei W die Gruppe -C(O)- oder eine Bindung bedeutet,

| | |
|---|---|
| A5 und A6 | gleich oder verschieden sind und -C(O)-, -NH-C(O)- oder eine Bindung bedeuten, |
| M | ausgewählt ist aus einer der nachfolgenden Gruppen |

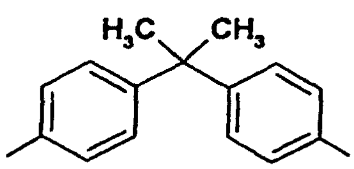 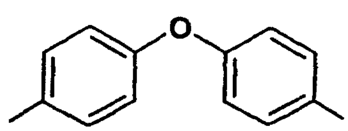 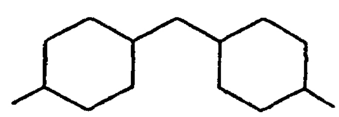

| | |
|---|---|
| K1 | -B7-(C(O))$_m$-B9-Z1-B11-X1 bedeutet, |
| K2 | -B8-(C(O))$_p$-B10-Z2-B12-X2 bedeutet, |
| B1, B2, B3, B4, B5 und B6 | gleich oder verschieden sind und eine Bindung oder -$CH_2$- (Methylen) bedeuten, |
| B7, B8, B9, B10, B11 und B12 | gleich oder verschieden sind und eine Bindung oder -$CH_2$- (Methylen) bedeuten, |
| m | 0 oder 1 bedeutet, |
| p | 0 oder 1 bedeutet, |
| X1 und X2 | gleich oder verschieden sind und Amino, Amidino oder Guanidino bedeuten, |
| Z1 und Z2 | gleich oder verschieden sind und 1,4-Phenylen, 1,3-Phenylen, 1,4-Cyclohexylen oder 1,4-Piperazinylen bedeuten, |

und worin auf direktem Weg zwischen den terminalen Stickstoffatomen 24 bis 40 Bindungen vorhanden sein müssen, die Salze dieser Verbindungen, wobei alle diejenigen Verbindungen ausgeschlossen sind bei denen eine oder mehrere der Variablen B1, B2, B3, B4, B5, B6, B7, B8, B9, B10, B11 oder B12 die Bedeutung einer Bindung annehmen und es dadurch zur direkten Verknüpfung zweier Heteroatome oder zweier Carbonylgruppen kommen würde.

Besonders bevorzugte Verbindungen sind Bis{4-[4-(4-aminomethylcyclohexanoyl)piperazin-1-yl]carbonyl}-4,4'-diamino-diphenylether, Bis{4-[(3-aminomethyl)benzoyl-piperazin-1-yl]carbonyl}4,4'-diamino-diphenylether, Di {4-[4-(4-aminomethyl)cyclohexanoylamino]piperidin-1-yl-carbamoyl}cyclohexylmethan, 2,2-Bis-[4-(4-guanidinyl-benzylamino)-carbonylmethoxyphenyl]propan, 2,2-Bis-[4-(10-amino-3,6-diaza-2,5-dioxodecyloxy)phenyl]-propan und 2,2-Bis-{4-[4-(4-aminomethylbenzylcarbamoyl)-1-piperazinyl-carbonyloxy]phenyl}propan, sowie die Salze dieser Verbindungen.

Erfindungsgemäß wird unter dem direkten Weg zwischen den Stickstoffatomen, die in den als X1' oder X2 definierten Gruppen als terminale Stickstoffatome fungieren, diejenige Anzahl von Bindungen angesehen, die durch Abzählen der Bindungen, die die kürzest mögliche Verbindungslinie zwischen den terminalen Stickstoffatomen darstellen, erhalten wird.

Folgendes Beispiel soll die Bestimmung der Anzahl der Bindungen auf dem direkten Weg zwischen zwei terminalen Stickstoffatomen verdeutlichen:

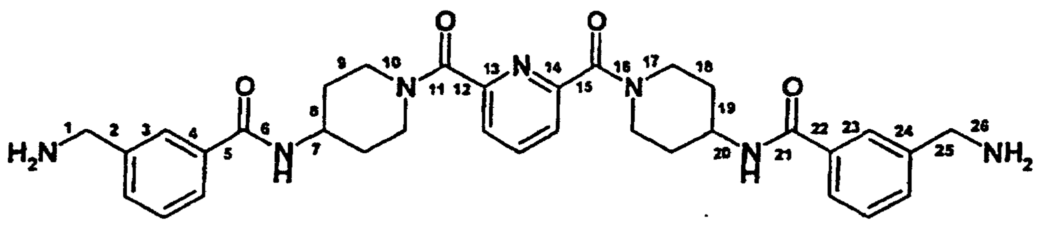

Der direkte Weg beinhaltet hier 26 Bindungen.

Bei den erfindungsgemäßen Inhibitoren handelt es sich um bifunktionelle Inhibitoren, d.h. Inhibitoren mit zwei bindefähigen, funktionellen Gruppen. Diese Gruppen sind derart ausgestaltet, daß sie spezifisch an aktive Stellen der Tryptase binden können. Bevorzugt binden die beiden funktionellen Gruppen des Inhibitors an aktive Stellen in verschiedenen Monomer-Untereinheiten des Tryptase-Tetramers.

Die erfindungsgemäßen Inhibitoren sind zur Hemmung von humaner Tryptase geeignet. Unter humaner Tryptase wird insbesondere das humane Enzym β-Tryptase mit der EC-Nr. 3.4.21.59 verstanden.

Als Salze kommen für Verbindungen der Formel I - je nach Substitution - alle Säureadditionssalze oder alle Salze mit Basen in Betracht. Besonders erwähnt seien die pharmakologisch verträglichen Salze der in der Galenik üblicherweise verwendeten anorganischen und organischen Säuren. Als solche eignen sich einerseits wasserlösliche und wasserunlösliche Säureadditionssalze mit Säuren wie beispielsweise Salzsäure, Bromwasserstoffsäure, Phosphorsäure, Salpetersäure, Schwefelsäure, Essigsäure, Zitronensäure, D-Gluconsäure, Benzoesäure, 2-(4-Hydroxybenzoyl)-benzoesäure, Buttersäure, Sulfosalicylsäure, Maleinsäure, Laurinsäure, Äpfelsäure, Fumarsäure, Bemsteinsäure, Oxalsäure, Weinsäure, Embonsäure, Stearinsäure, Toluolsulfonsäure, Methansulfonsäure oder 3-Hydroxy-2-naphthoesäure, wobei die Säuren bei der Salzherstellung - je nachdem, ob es sich um eine ein- oder mehrbasige Säure handelt und je nachdem, welches Salz gewünscht wird - im äquimolaren oder einem davon abweichenden Mengenverhältnis eingesetzt werden.

Andererseits kommen auch Salze mit Basen in Betracht. Als Beispiele für Salze mit Basen seien Alkali- (Lithium-, Natrium-, Kalium-) oder Calcium-, Aluminium-, Magnesium-, Titan-, Ammonium-, Meglumin- oder Guanidiniumsalze erwähnt, wobei auch hier bei der Salzherstellung die Basen im äquimolaren oder einem davon abweichenden Mengenverhältnis eingesetzt werden.

Pharmakologisch unverträgliche Salze, die beispielsweise bei der Herstellung der erfindungsgemäßen Verbindungen im industriellen Maßstab als-Verfahrensprodukte zunächst anfallen können, werden durch dem Fachmann bekannte Verfahren in pharmakologisch verträgliche Salze übergeführt.

Dem Fachmann ist bekannt, daß die erfindungsgemäßen Verbindungen als auch ihre Salze, wenn sie zum Beispiel in kristalliner Form isoliert werden, verschiedene Mengen an Lösungsmitteln enthalten können. Die Erfindung umfaßt daher auch alle Solvate und insbesondere alle Hydrate der Verbindungen der Formel I, sowie alle Solvate und insbesondere alle Hydrate der Salze der Verbindungen der Formel I.

Die Verbindungen der Formel I setzen sich aus einer Vielzahl divalenter Bausteine (M, A1, A2, A3, A4, A5, A6, B1, B2, B3, B4, B5, B6)[-] zusammen. Ihre Synthese kann grundsätzlich ausgehend von jedem dieser Bausteine erfolgen. Bei weitgehend symmetrisch aufgebauten Verbindungen der Formel I ist der Aufbau beginnend vom Zentralbaustein M bevorzugt, während bei überwiegend unsymmetrischen Verbindungen der Formel I die Synthese ausgehend von einer der Endgruppen K1 oder K2 vorteilhaft sein kann.

Die Verknüpfung der Bausteine erfolgt dabei immer nach dem gleichen, dem Fachmann an sich bekannten Muster.

Dem Fachmann ist bekannt, daß die Verbindungen der Formel I entweder Baustein für Baustein aufgebaut werden können, oder daß zunächst größere aus mehreren Einzelbausteinen bestehende Fragmente erstellt werden können, die anschließend zum Gesamtmolekül zusammengesetzt werden.

Aufgrund der Bedeutungen, die die einzelnen Bausteine der Verbindungen der Formel I annehmen können, treten in den Verbindungen der Formel I Amino- [-NH-], Ether [-O-], Keto- [-C(O)-], Ester- [-O-C(O)-, -C(O)-O-], Amid-[-C (O)-NH-, -NH-C(O)-], Carbamat- [-NH-C(O)-O-, -O-C(O)-NH-], Carbamid- (-NH-C(O)-NH-) oder Carbonatbrücken [-O-C(O)-O-] auf.

Die Art und Weise, wie solche Brücken hergestellt werden, sind dem Fachmann an sich bekannt, geeignete Methoden und Ausgangsverbindungen zu ihrer Herstellung werden beispielsweise in March, Advanced Organic Chemistry , Reactions, Mechanisms and Structure, Third Edition, 1985, John Wiley & Sons beschrieben.

Ether- brücken können beispielsweise nach der Methode von Williamson hergestellt werden.

Keto- brücken können beispielsweise als Bestandteil größerer Bausteine, wie z. B. dem 1,3-Dichloraceton eingeführt werden.

Für den Aufbau von Esterbrücken ist eine Vielzahl von Methoden bekannt. Beispielhaft genannt sei hier die Umsetzung von Säuren mit Alkoholen, vorzugsweise unter Verwendung von $H_2SO_4$ oder p-Toluolsulfonsäure als Katalysator; oder unter Zugabe eines wasserentziehenden Mittels, wie zum Beispiel Molekularsieb oder einem Carbodiimid. Desweiteren kann hier die Umsetzung von Säurechloriden mit Alkoholen genannt werden.

Auch für die Darstellung von Amidbrücken gibt es eine Vielzahl bekannter Methoden. Als Beispiel sei hier die Umsetzung von Säurechloriden mit primären oder sekundären Aminen genannt. Desweiteren sei auch auf all die Methoden verwiesen, die für die Peptidchemie entwikkelt wurden. Entsprechend lassen sich aus Sulfonsäurechloriden und primären oder sekundären Aminen Sulfonamidbrücken aufbauen.

Carbamatbrücken können z. B. durch Reaktion von Chlorkohlensäureestern mit Aminen hergestellt werden. Die Chlorkohlensäureester ihrerseits können aus Alkoholen und Phosgen aufgebaut werden.

Eine weitere Variante zum Aufbau von Carbamatbrücken stellt die Addition von Alkoholen an Isocyanate dar.

Ähnlich wie bei den Carbamatbrücken können ausgehend von Chlorkohlensäureestern durch Umsetzung mit Alkoholen (anstatt Aminen) Carbonatbrücken hergestellt werden.

Carbamidbrücken lassen sich z. B. durch die Reaktion von Isocyanaten mit Aminen herstellen.

Verbindungen der Formel I können auch durch Derivatisierung in weitere Verbindungen der Formel I übergeführt werden. So können beispielsweise Verbindungen der Formel I, die einen ein Stickstoffatom enthaltenden Heteroaryl- oder Heterocycloalkylbaustein aufweisen durch Oxidation in die entsprechenden N-Oxide übergeführt werden.

Die N-Oxidation erfolgt auf eine dem Fachmann ebenfalls vertraute Weise, z.B. mit Hilfe von Wasserstoffperoxid in Methanol oder m-Chlorperoxibenzoesäure in Dichlormethan bei Raumtemperatur. Welche Reaktionsbedingungen für die Durchführung des Verfahren im einzelnen erforderlich sind, ist dem Fachmann aufgrund seines Fachwissens geläufig.

Dem Fachmann ist außerdem bekannt, daß es im Fall mehrerer reaktiver Zentren an einer Ausgangs- oder Zwischenverbindung notwendig sein kann, ein oder mehrere reaktive Zentren temporär durch Schutzgruppen zu blokkieren, um eine Reaktion gezielt am gewünschten Reaktionszentrum ablaufen zu lassen. Eine ausführliche Beschreibung zur Anwendung einer Vielzahl bewährter Schutzgruppen findet sich beispielsweise in T.W. Greene, Protective Groups in Organic Synthesis, John Wiley & Sons, 1991.

Die Isolierung und Reinigung der erfindungsgemäßen Substanzen erfolgt in an sich bekannter Weise z.B. derart, daß man das Lösungsmittel im Vakuum abdestilliert und den erhaltenen Rückstand aus einem geeigneten Lösungsmittel umkristallisiert oder einer der üblichen Reinigungsmethoden, wie beispielsweise der Säulenchromatographie an geeignetem Trägermaterial, unterwirft.

Salze erhält man durch Auflösen der freien Verbindung in einem geeigneten Lösungsmittel, z.B. in einem chlorierten Kohlenwasserstoff, wie Methylenchlorid oder Chloroform, oder einem niedermolekularen aliphatischen Alkohol (Ethanol, Isopropanol), das die gewünschte Säure bzw. Base enthält, oder dem die gewünschte Säure bzw. Base anschließend zugegeben wird. Die Salze werden durch Filtrieren, Umfällen, Ausfällen mit einem Nichtlösungsmittel für das Anlagerungssalz oder durch Verdampfen des Lösungsmittels gewonnen. Erhaltene Salze können durch Alkalisierung bzw. durch Ansäuern in die freien Verbindungen umgewandelt werden, weiche wiederum in Salze übergeführt werden können. Auf diese Weise lassen sich pharmakologisch nicht verträgliche Salze in pharmakologisch verträgliche Salze umwandeln.

Die Herstellung von Verbindungen der Formel I sei exemplarisch an Hand der nachfolgenden Beispiele 1 bis 12 und der Figuren 1 bis 12 aufgezeigt. Weitere Verbindungen der Formel I können analog oder unter Anwendung der oben aufgeführten, dem Fachmann an sich bekannten . Methoden hergestellt werden.

Die Figuren 6, 7, 8, 9, 10, 11 und 12 zeigen Formelschemata für die Herstellung von erfindungsgemäßen

bifunktionellen Inhibitoren.

**Beispiel 1:**

ENDPRODUKT:

**Bis{4-[4-(4-aminomethyl)cyclohexanoyl)piperazin-1-yl]carbonyl}4,4'-diamino-diphenylether Dihydrochlorid (17)** (vgl. Fig. 6)

Bis{4-[4-(4-tert-butoxycarbonyl-aminomethyl)cyclohexanoyl-piperazin-1-yl]carbonyl}4,4'-diamino-diphenylether (0,18 g; 0,2 mmol) wird in 4,8 M HCl in Dioxan (5 ml) suspendiert. Die Suspension wird 24 Stunden bei 40 - 45°C gerührt. Nach Zugabe von Diethylether (25 ml) wird im Eisbad gekühlt. Das ausgefallene Produkt wird abgenutscht, mehrmals mit Diethylether gewaschen und im Vakuum getrocknet. Ausbeute: 0,12 g, weißer amorpher Feststoff. MS (ESI): 703,4 (100) $MH^+$

AUSGANGSVERBINDUNGEN:

**Bis{4-[4-(4-tert-butoxycarbonyl-aminomethyl)cyclohexanoyl-piperazin-1-yl]carbonyl}4,4'diamino-diphenylether (18)**

4,4'-Bis(1-piperazinylcarbamoyl)diphenylether-dihydrochlorid (0,25 g; 0,5 mmol), Boc-tranexamsäure (0,28 g; 1,1 mmol), N-Ethyldiisopropylamin (0,2 ml; 1,1 mmol) und 4-Dimethylaminopyridin (5 mg) werden in Dimethylformamid (2,5 ml) und Dichlormethan (2,5 ml) 15 Minuten bei Raumtemperatur gerührt. Nach Zugabe von N-(3-Dimethylaminopropyl)-N'-etyhlcarbodiimid-hydrochlorid (0,21 g; 1,1 mmol) wird das Reaktionsgemisch 24 Stunden bei 40°C gerührt. Das Lösungsmittel wird im Vakuum vollständig abgezogen. Der Rückstand wird an Kieselgel chromatographiert (Dichlormethan : Methanol - 9 : 1). Die Produktfraktion wird gesammelt und das Lösungsmittel vollständig im Vakuum abgezogen. Ausbeute: 0,18 g, weißer amorpher Feststoff.
MS (ESI): 903,1 (100) $MH^+$

**4,4'-Bis(1-piperazinylcarbamoyl)diphenylether Dihydrochlorid (19)**

4,4'-Bis[4-(tert-butyloxycarbonyl)-1-piperazinylcarbamoyl]diphenylether (6,4 g; 10,2 mmol) wird in 4,8 M HCl in Dioxan (50 ml) suspendiert. Die Suspension wird 22 Stunden bei 40-45°C gerührt. Nach Zugabe von Diethylether (100 ml) wird im Eisbad gekühlt. Das ausgefallene Produkt wird abgenutscht, mehrmals mit Diethylether gewaschen und im Vakuum getrocknet. Ausbeute: 4,65 g, weißer amorpher Feststoff.
MS(APCI): 425,0 (100) $MH^+$

**4,4'-Bis[4-(tert-butyloxycarbonyl)-1-piperazinylcarbamoyl]diphenylether (20)**

Zur gerührten Lösung von 1-tert.-Butoxycarbonylpiperazin (4,10 g: 22 mmol) in Dichlormethan (50 ml) wird bei Raumtemperatur eine Lösung von Oxy-bis-(4-phenyl-isocyanat) (2,52 g , 10 mmol) in Dichlormethan (25 ml) zugetropft. Nach beendeter Zugabe wird weitere drei Stunden bei Raumtemperatur gerührt. Das ausgefallene Produkt wird abgenutscht, mehrmals mit Hexan gewaschen und in Vakuum getrocknet. Ausbeute: 6,20 g weißer amorpher Feststoff.
MS(EI): 625,5 (12) $MH^+$; 271,2 (26); 118,2 (42); 187,1 (100)

**Beispiel 2:**

ENDPRODUKT:

**Bis{4-[4-(3-aminomethyl)benzoyl-piperazin-1-yl]carbonyl]4,4'-diaminodiphenylether Dihydrochlorid (21)** (vgl. Fig. 7)

Bis{4-[4-(3-tert-butoxycarbonyl-aminomethyl)benzoyl-piperazin-1-yl]carbonyl}4,4'-diamino-diphenylether (0,31 g; 0,35 mmol) wird in 4,8 M HCl in Dioxan (5 ml) 24 Stunden bei 40 - 45°C gerührt. Nach Zugabe von Diethylether (25 ml) wird im Eisbad gekühlt. Das ausgefallene Produkt wird abgenutscht, mehrmals mit Diethylether gewaschen und im Vakuum getrocknet. Ausbeute: 0,19 g, weißer amorpher Feststoff.
MS (ESI): 691.2 (100) $MH^+$

AUSGANGSVERBINDUNGEN:

**Bis{4-[4-(3-tert-butoxycarbonyl-aminomethyl)benzoyl-piperazin-1-yl]carbonyl}-4,4'-di-amino-diphenylether (22)**

4,4'-Bis(1-piperazinylcarbamoyl)diphenylether Dihydrochlorid (0,25 g; 0,5 mmol), 3-(tert.-butoxycarbonylaminomethyl)benzoesäure (0,28 g; 1,1 mmol), N-Ethyldiisopropylamin (0,2 ml; 1,1 mmol) und 4-Dimethylaminopyridin (30 mg) werden in Dimethylformamid (2,5 ml) und Dioxan (2,5 ml) 15 Minuten bei Raumtemperatur gerührt. Nach Zugabe von N-(3-Dimethylaminopropyl)-N'-etyhlcarbodiimid-hydrochlorid (0,21 g; 1,1 mmol) wird das Reaktionsgemisch 24 Stunden bei Raumtemperatur gerührt. Das Lösungsmittel wird im Vakuum vollständig abgezogen. Der Rückstand wird an Kieselgel chromatographiert (Dichlormethan : Methanol - 9 : 1). Die Produktfraktion wird gesammelt und das Lösungsmittel vollständig im Vakuum abgezogen. Ausbeute: 0,32 g, viskoses Öl.
MS (ESI): 890.8, $M^+$; 791.2, $MH\text{-}Boc^+$

**Beispiel 3:**

ENDPRODUKT:

**Di{4-[4-(4-aminomethyl)cyclohexanoylamino]piperidin-1-yl-carbamoyl}cyclohexylmethan Dihydrochlorid (23)** (vgl. Fig. 8)

Di{4-[4-(4-tert.-butoxycarbonyl-aminomethyl)cyclohexanoylamino]piperidin-1-yl-carbamoyl}cyclohexylmethan (0,65 g; 0,7 mmol) wird in 4,8 M HCl in Dioxan (7 ml) 24 Stunden bei 40 - 45°C gerührt. Nach Zugabe von Diethylether (50 ml) wird im Eisbad gekühlt. Das ausgefallene Produkt wird abgenutscht, mehrmals mit Diethylether gewaschen und im Vakuum getrocknet. Ausbeute: 0,26 g, weißer amorpher Feststoff.
MS (ESI): 741,5 (100) $MH^+$

AUSGANGSVERBINDUNGEN:

**Di{4-[4-(4-tert.-butoxycarbonyl-aminomethyl)cyclohexanoylamino]piperidin-1-yl-carbamoyl}cyclohexylmethan (24)**

Di[4-(4-Amino-piperidin-1-yl-carbamoyl)]cyclohexyl-methan Dihydrochlorid (0,54 g; 1,0 mmol), Boc-tranexamsäure (0,57 g; 2,2 mmol), N-Ethyldiisopropylamin (0,38 ml; 2,2 mmol) und 4-Dimethylaminopyridin (30 mg) werden in Dimethylformamid (5 ml) und Dioxan (5 ml) 15 Minuten bei Raumtemperatur gerührt. Nach Zugabe von N-(3-Dimethylaminopropyl)-N'-ethylcarbodiimidhydrochlorid (0,43 g; 2,2 mmol) wird das Reaktionsgemisch 48 Stunden bei 40°C gerührt. Das Lösungsmittel wird im Vakuum vollständig abgezogen. Der Rückstand wird an Kieselgel chromatographiert (Dichlormethan : Methanol - 9 : 1). Die Produktfraktion wird gesammelt und das Lösungsmittel vollständig im Vakuum abgezogen. Ausbeute: 0,65 g, viskoses Öl, das ohne Charakterisierung weiter umgesetzt wurde.

**Di[4-(4-Amino-piperidin-1-yl-carbamoyl)]cyclohexyl-methan Dihydrochlorid (25)**

Di{4-[4-(tert.-Butoxycarbamoyl)piperidin-1-yl-carbamoyl]}cyclohexyl-methan (4,90 g; 7,0 mmol) wird in 4,8 M HCl in Dioxan (50 ml) suspendiert. Die Suspension wird 48 Stunden bei 40 - 45°C gerührt. Nach Zugabe von Diethylether (100 ml) wird im Eisbad gekühlt. Das ausgefallene Produkt wird abgenutscht, mehrmals mit Diethylether gewaschen und im Vakuum getrocknet. Ausbeute: 4,10 g, weißer amorpher Feststoff.
MS(EI): 463,4 (100) $MH^+$

**Di{4-[4-(tert.-Butoxycarbamoyl)piperidin-1-yl-carbamoyl]}cyclohexyl-methan (26)**

Zur gerührten Lösung von 4-tert.-Butoxycarbamoyl-piperidin (3,20 g; 16,0 mmol) in Dichlormethan (30 ml) wird bei Raumtemperatur eine Lösung von Dicyclohexylmethan-4,4'-diisocyanat (1,90 g ; 7,3 mmol) in Dichlormethan (10 ml) zugetropft. Nach beendeter Zugabe wird weitere drei Stunden bei Raumtemperatur gerührt. Das ausgefallene Produkt wird abgenutscht, mehrmals mit Hexan gewaschen und in Vakuum getrocknet. Ausbeute: 4,10 g weißer amorpher Feststoff.
MS(ESI): 685,3 (57) $MNa^+$; 663,2 (100) $MH^+$

**Beispiel 4:**

ENDPRODUKT:

**2,2-Bis{4-[4-(4-aminophenyl)-1-piperazinylcarbonyl-methoxy]phenyl}propan Dihydrochlorid (27)** (vgl. Fig. 9)

0,65 g 2,2-Bis{4-[4-(4-nitrophenyl)-1-piperazinylcarbonyl-methoxy]phenyl}propan werden in 60 ml Eisessig gelöst und 0,2 g Palladiumkohle (10 %) zugegeben. Das Gemisch wird in einer Umlaufapparatur hydriert bis kein Ausgangsprodukt mehr nachweisbar ist (DC). Es wird vom Katalysator über Celite abgesaugt und das Filtrat am Rotationsverdampfer im Vakuum bis zur Trocknung eingedampft. Der Rückstand wird im Dichlormethan gelöst, die Lösung mit $NaHCO_3$-Lösung gewaschen, über $Na_2SO_4$ getrocknet, filtriert und wieder eingeengt. Der Rückstand wird über eine Kieselgelsäule mit einem Gemisch aus Ethylacetat/Methanol/$NH_4OH$ (25 %) im Verhältnis von 90:8:2 als Laufmittel chromatographiert. Die chromatographisch reinen Fraktionen werden vereint, eingeengt und der Rückstand in Dichlormethan gelöst. Nach Zugabe von ätherischer Salzsäure wird eingeengt, noch zweimal mit Dichlormethan nachdestilliert und dann der Rückstand mit Ethylacetat/Isopropanol verrieben. Der Niederschlag wird abgesaugt, gewaschen und dann im Hochvakuum getrocknet. Man erhält 0,32 g der Titelverbindung mit Schmp. ab 182 °C Zersetzung.

AUSGANGSVERBINDUNGEN:

**2,2-Bis{4-[4-(4-nitrophenyl)-1-piperazinylcarbonylmethoxy]phenyl}propan (28)**

2,5 g 4-[4-Carboxylmethoxyphenyl)-1-methyl-ethyl]phenoxyessigsäure werden in Toluol suspendiert und 1,6 ml Thionylchlorid zugegeben. Das Gemisch wird 5 Stunden unter Rückfluß erhitzt und nach Abkühlen am Rotationsverdampfer eingeengt. Es wird noch zweimal mit Toluol nachdestilliert und dann das erhaltene rohe Disäurechlorid in 50 ml abs. Dioxan gelöst. Es werden nacheinander 2,95 g 1-(4-Nitrophenyl)-piperazin, 2 ml Triethylamin und eine Spatelspitze 4-Dimethylaminopyridin zugegeben. Das Gemisch wird 2,5 h bei 50 °C gerührt. Nach Abkühlen wird mit Wasser versetzt, der pH mit verdünnter Natronlauge auf 9 eingestellt. Das abgeschiedene Produkt wird durch Anreiben zur Kristailisation gebracht, abgesaugt, mit Wasser gewaschen und über Calciumchlorid getrocknet. Man erhält 4,7 g der Titelverbindung mit Schmp. ab 165 °C Zersetzung.

**4-[1-(4-Carboxymethoxyphenyl)-1-methyl-ethyl]-phenoxyessigsäure (29)**

6,7 g 4-[1-(4-Ethaxycarbonylmethoxyphenyl)-1-methyl-ethyl]phenoxyessigsäure-ethylester werden in 20 ml Methanol gelöst und 16,7 g 10 %-ige Natronlauge zugegeben. Das Gemisch wird 3 Stunden unter Rückfluß zum Sieden erhitzt, abgekühlt und dann das Methanol am Rotationsverdampfer abdestilliert. Es wird mit Wasser verdünnt, mit 2 N HCl auf pH 2 angesäuert und dann der farblose Niederschlag abgesaugt, mit Wasser gewaschen und im Vakuum über Calciumchlorid getrocknet. Man enthält 5,5 g der Titelverbindung mit Schmp. 177 - 179 °C.

**4-[1-(4-Ethoxycarbonylmethoxyphenyl)-1-methyl-ethyl]phenoxyessigsäureethylester (30)**

Ein Gemisch aus 10 g 4,4'-Isopropylidendiphenol, 10,7 ml Bromessigsäureethylester, 15,2 g Kaliumcarbonat und 1 Spatelspitze 18-Krone-6 in 180 ml Aceton wird in 4 Stunden unter Rückfluß zum Sieden erhitzt. Dann wird vom Feststoff abgesaugt, das Filtrat im Vakuum eingeengt und der Rückstand mit 100 ml Diisopropylether versetzt. Es wird abgesaugt, mit wenig Diisopropylether gewaschen und getrocknet. Man erhält 15,5 g der Titelverbindung mit Schmp. 69 - 71 °C.

**Beispiel 5:**

ENDPRODUKT:

**2,2-Bis-[4-(4-guanidinyl-benzylamino)carbonylmethoxyphenyl]propan-dihydroacetat (31)** (vgl. Fig. 10)

0,63 g 2,2-Bis-[4-(4-aminobenzylamino)carbonylmethoxyphenyl]propan in 10 ml abs. DMF werden nacheinander unter Rühren mit 0,88 g 1,3-Bis(benzyloxycarbonyl)-2-methylisothiohamstoff, 0,68 g Quecksilber(II)chlorid und 0,69 g Triethylamin versetzt. Das Gemisch wird 3 h bei Raumtemperatur gerührt, dann wird mit Ethylacetat verdünnt, vom entstandenen Niederschlag abgesaugt und das Filtrat einmal mit 5 %iger Sodalösung und zweimal mit Wasser gewaschen. Die Lösung wird über Magnesiumsulfat getrocknet, abgesaugt und das Filtrat im Vakuum zur Trockene eingedampft. Das Öl wird über eine Kieselgelsäule mit einem Gemisch aus Dichlormethan/Ethanol 95:5 chromatografiert. Die chromatografisch reinen Franktionen werden vereinigt, eingeengt und der Rückstand (0,9 g) in einem Gemisch aus 60 ml Tretrahydrofuran, 3 ml Methanol und 1 ml Eisessig gelöst. Nach Zugabe von 0,3 g Palladiumkohle (10 %) wird in einer Umlaufapparatur hydriert bis kein Ausgangsprodukt mehr nachweisbar ist. Es wird vom Katalysator abgesaugt und zur Trockene eingedampft. Das verbleibende zähe Öl wird mit THF verrührt, der entstandene Niederschlag abgesaugt, mit THF und Diethylether gewaschen und in Vakuum bei 80° C getrocknet. Man erhält 0,35 g der Titelverbindung mit Schmp. 135° (Zersetzung).

**AUSGANGSVERBINDUNGEN:**

**2,2-Bis-[4-(4-aminobenzylamino)carbonylmethoxyphenyl]propan (32)**

1,8 g 2,2-Bis-[4-(4-nitrobenzylamino)carbonylmethoxyphenyl]propan werden in 300 ml THF gelöst und nach Zugabe von 0,5 g Palladiumkohle (10 %) in einer Umlaufapparatur hydriert bis kein Ausgangsprodukt mehr nachweisbar ist (DC). Nach Absaugen des Katalysators wird das Filtrat im Vakuum zur Trockene eingeengt und der Rückstand über eine Kieselgelsäule mit einem Gemisch aus Dichlormethan/Ethanol 95:5 chromatografiert. Die chromatografisch reinen Fraktionen werden vereint, eingeengt und der Rückstand im Hochvakuum getrocknet. Man erhält 1,05 g der Titelverbindung in Form eines erstarrten Schaumes.

**2,2-Bis-[4-(4-nitrobenzylamino)carbonylmethoxyphenyl]propan (33)**

2 g 4-[1-(4-Carboxymethoxyphenyl)-1-methylethyl]-phenoxyessigsäure in 100 ml Toluol werden mit 1,5 ml Thionylchlorid versetzt und das Gemisch 5 h unter Rückfluß zum Sieden erhitzt. Nach Abkühlen wird am Rotationsverdampfer eingeengt und noch zweimal mit Toluol nachdestilliert. Das so erhaltene Disäurechlorid wird in 40 ml abs. Dioxan gelöst, 2,2 g 4-Nitrobenzylamin-hydrochlorid zugegeben und dann 3,5 ml Triethylamin zugetropft. Das Gemisch wird 2 h bei 50 °C gerührt und dann im Vakuum eingeengt. Der nach Zugabe von Wasser entstandene Niederschlag wird abgesaugt, im Vakuum getrocknet und zur weiteren Reinigung über eine Kieselgelsäule mit Ethylacetat chromatografiert. Die chromatografisch reinen Fraktionen werden vereint, eingeengt und getrocknet. Man erhält 1,25 g der Titelverbindung als erstarrten Schaum.

**Beispiel 6:**

ENDPRODUKT:

**2,2-Bis-[4-(10-amino-3,6-diaza-2,5-dioxodecyloxy)phenyl]propan-dihydrochlorid (34)** (vgl. Fig. 11)

0,77 g 2,2-Bis-{4-[10-(tert.butoxycarbonylamino)-3,6-diaza-2,5-dioxodecyloxy]phenyl}propan werden in 10 ml abs. Dioxan gelöst und mit 2 ml einer ca. 4,8 M Lösung von Chlorwasserstoff in Dioxan versetzt. Es wird über Nacht gerührt, dann der entstandene Niederschlag abgesaugt, mit Dioxan und dann mit Diethylether gewaschen und bei 80° C im Vakuum getrocknet. Man erhält 0,58 g der Titelverbindung mit Schmp. 173° C (Zersetzung).

AUSGANGSVERBINDUNGEN:

**2,2-Bis-[4-{10-(tert.butoxycarbonylamino)-3,6-diaza-2,5-dioxodecyloxy]phenyl}propan (35)**

0,67 g 2,2-Bis-(4-chlorcarbonylmethoxyphenyl)propan (hergestellt analog Beispiel 33) in 5 ml abs. Dioxan werden unter Rühren zu einer Lösung von 0,85 g N-[4-(tert.Butoxycarbonylamino)butyl]glycinamid und 0,42 Triethylamin in 10 ml abs. Dioxan zugetropft. Die Mischung wird über Nacht gerührt, in Vakuum eingeengt und der Rückstand zwischen Wasser und Ethylacetat verteilt. Die organische Phase wird zweimal mit Wasser gewaschen, Ober Magnesiumsulfat getrocknet und eingeengt. Der Rückstand wird über eine Kieselgelsäule mit einem Gemisch aus Dichlormethan/Ethanol 95:5 chromatografiert. Die chromatografisch reinen Fraktionen werden vereint, eingeengt und der Rückstand mit Diethylether/2-Propanol kristallisiert. Es wird abgesaugt, mit Diethylether gewaschen und in Vakuum getrocknet. Man erhält 0,77 g der Titelverbindung mit Schmp. 59° C (Zersetzung).

**Beispiel 7:**

ENDPRODUKT:

**2,2-Bis-{4-[4-(4-aminomethylbenzylcarbamoyl)-1-piperazinylcarbonyloxy]phenyl}propandihydrochlorid (36)** (vgl. Fig. 12)

0,14 g 2,2-Bis-{4-[4-(4-tert.butoxycarbonylaminomethylbenzylcarbamoyl)-1-piperazinylcarbonyloxy]phenyl) propan werden in 2 ml abs. Dioxan gelöst und mit 2 ml einer ca. 20%igen Chlorwasserstoff-Lösung im Dioxan versetzt. Es wird Ober Nacht gerührt, abgesaugt, zweimal mit Diethylether gewaschen und im Vakuum getrocknet. Man erhält 0,08 g der Titelverbindung mit Schmp. ab 250° C (Zersetzung).

AUSGANGSVERBINDUNGEN:

**2,2-Bis-{4-[4-(4-tert.butoxycarbonylaminomethylbenzylcarbamoyl]-1-piperazinylcarbonyloxy]phenyl)propan (37)**

0,2 g 2,2-Bis-[4-(1-piperazinylcarbonyloxy)phenyl]propan-dihydrochlorid und 0,66 ml Diisopropylethylamin werden in 5 ml Dichlormethan gelöst und dann mit 0,4 ml einer 20%igen Phosgenlösung in Toluol versetzt. Nach 30 min Rühren bei Raumtemperatur werden 0,18 g 4-(tert.Butoxycarbonylaminomethyl)benzylamin zugegeben und weitere 30 min. gerührt. Dann wird mit Wasser versetzt, die Phasen getrennt und die organische Phase noch zweimal mit Wasser gewaschen. Nach Trocknen über Magnesiumsulfat wird am Rotationsverdampfer eingeengt. Der Rückstand wird Ober eine Kieselgelsäule mit Dichlormethan/Methanol 95:5 als Laufmittel chromatografiert. Die chromatografisch reinen Fraktionen werden vereinigt und im Vakuum zur Trockene eingedampft. Man erhält 0,17 g der Titelverbindung als erstarrten Schaum.

**2,2-Bis-[4-(1-piperazinylcarbonyloxy)phenyl]propan-dihydrochlorid (38)**

8,3 g 2,2-Bis-[4-(4-tert.butoxycarbonyl-1-piperazinylcarbonyloxy)phenyl]propan-dihydrochlorid werden in 50 ml abs. Dioxan gelöst und unter Rühren mit 9,5 ml einer ca. 20%igen Chlorwasserstofflösung in Dioxan versetzt. Das Gemisch wird über Nacht gerührt, mit Toluol verdünnt und der Niederschlag abgesaugt. Nach Trocknen im Vakuum erhält man 5,7 g der Titelverbindung mit Schmp. ab 200° C (Zersetzung).

**2,2-Bis-[4-(4-tert.butoxycarbonyl-1-piperazinylcarbonyloxy)phenyl]propan (39)**

5 g Bisphenol A-bis(chloroformat) werden in 50 ml Dichlormethan gelöst und unter Eiskühlung 7,3 ml Diisopropylethylamin und 6,6 g 1-tert.Butoxycarbonylpiparazin zugegeben. Die Mischung wird 1 h bei Raumtemperatur gerührt und dann dreimal mit eiskalter 0,5 N Salzsäurelösung und zweimal mit 1 N Natronlauge extrahiert. Nach Trocknen mit Magnesiumsulfat wird am Rotationsverdampfer eingedampft und der Feststoff im Vakuum getrocknet. Man erhält 8,4 g der Titelverbindung mit Schmp. 171-172 °C.

**Gewerbliche Anwendbarkeit**

Die erfindungsgemäßen Verbindungen besitzen als Inhibitoren der humanen Tryptase wertvolle pharmakologische Eigenschaften, die sie gewerblich verwertbar machen. Humane Tryptase ist eine Serinprotease, die in humanen Mastzellen das überwiegend vorliegende Protein darstellt. Tryptase umfaßt vier eng verwandte Enzyme (α, I, II/β, III; 90 bis 98 % Sequenzidentität) (vgl. Miller et al., J. Clin. Invest. 84 (1989) 1188-1195; Miller et al., J. Clin. Invest. 86 (1990) 864-870; Vanderslice et al., Proc. Natl. Acad. Sci., USA 87 (1990) 3811-3815). Mit Ausnahme der α-Tryptase (Schwartz et al., J. Clin. Invest. 96 (1995) 2702-2710; Sakai et al., J. Clin. Invest. 97 (1996) 988-995) werden die Enzyme intrazellulär aktiviert und in katalytisch aktiver Form in Sekretgranulen gelagert. Tryptase weist im Vergleich zu anderen bekannten Serinproteasen, wie zum Beispiel Trypsin oder Chymotrypsin einige besondere Eigenschaften auf (Schwartz et al., Methods Enzymol. 244, (1994), 88-100; G. H. Caughey, "Mast cell proteases in immunology and biology". Marcel Dekker, Inc., New York, 1995). Tryptase aus humanen Gewebe weist eine nicht kovalent verknüpfte tetramere Struktur auf, die durch Heparin oder andere Proteoglycane stabilisiert sein muß, um proteolytisch aktiv zu sein. Tryptase wird zusammen mit anderen Entzündungsmediatoren, wie z. B. Histamin und Proteoglycanen, freigesetzt, wenn humane Mastzellen aktiviert werden. Man vermutet deshalb, daß Tryptase bei einer Reihe von Erkrankungen, insbesondere bei allergischen und entzündlichen Erkrankungen eine Rolle spielt, zum einen aufgrund der Bedeutung der Mastzellen bei solchen Erkrankungen und zum anderen, da bei einer Reihe derartiger Erkrankungen ein

erhöhter Tryptase-Gehalt festgestellt wurde. So wird Tryptase u. a. mit folgenden Krankheiten in Zusammenhang gebracht: Akute und chronische (insbesondere entzündliche und allergen induzierte) Atemwegserkrankungen verschiedener Genese ( z. B. Bronchitis, allergische Bronchitis, Asthma bronchiale, COPD); interstitielle Lungenerkrankungen, Erkrankungen, die auf allergischen Reaktionen der oberen Atemwege (Rachenraum, Nase) und der angrenzenden Regionen (z. B. Nasennebenhöhlen, Augenbindehäute) beruhen, wie beispielsweise allergische Konjunktivitis und allergische Rhinitis; Erkrankungen aus dem Formenkreis der Arthritis (z. B. rheumatische Arthritis); AutoimmunErkrankungen wie Multiple Sklerose; desweiteren Periodontitis, Anaphylaxis, interstitiale Cystitis, Dermatitis, Psoriasis, Sklerodermie/systemische Sklerose, entzündliche Darmerkrankungen (Morbus Crohn, Inflammatory Bowel Disease) und andere. Tryptase scheint insbesondere direkt mit der Pathogenese von Asthma in Zusammenhang zu stehen (Caughey, Am. J. Respir. Cell Mol. Biol. 16 (1997). 621-628; R. Tanaka, "The role of tryptase in allergic inflammation" in: Protease Inhibitors, IBC Library Series, 1979, Kapitel 3.3.1-3.3.23).

Weiterer Gegenstand der Erfindung sind die erfindungsgemäßen Verbindungen zur Anwendung bei der Behandlung und/oder Prophylaxe von Krankheiten, insbesondere den genannten Krankheiten.

Ebenso betrifft die Erfindung die Verwendung der erfindungsgemäßen Verbindungen zur Herstellung von Arzneimitteln, die zur Behandlung und/oder Prophylaxe der genannten Krankheiten eingesetzt werden.

Weiterhin sind Arzneimittel zur Behandlung und/oder Prophylaxe der genannten Krankheiten, die eine oder mehrere der erfindungsgemäßen Verbindungen enthalten, Gegenstand der Erfindung.

Die Arzneimittel werden nach an sich bekannten, dem Fachmann geläufigen Verfahren hergestellt. Als Arzneimittel werden die erfindungsgemäßen Verbindungen (= Wirkstoffe) entweder als solche, oder vorzugsweise in Kombination mit geeigneten pharmazeutischen Hilfsstoffen z.B. in Form von Tabletten, Dragees, Kapseln, Suppositorien, pflastern, Emulsionen, Suspensionen, Gelen oder Lösungen eingesetzt, wobei der Wirkstoffgehalt vorteilhafterweise zwischen 0,1 und 95 % beträgt.

Welche Hilfsstoffe für die gewünschten Arzneiformulierungen geeignet sind, ist dem Fachmann aufgrund seines Fachwissens geläufig. Neben Lösemitteln, Gelbildnern, Salbengrundlagen und anderen Wirkstoffträgern können beispielsweise Antioxidantien, Dispergiermittel, Emulgatoren, Konservierungsmittel, Lösungsvermittler oder Permeationspromotoren verwendet werden.

Für die Behandlung von Erkrankungen des Respirationstraktes werden die erfindungsgemäßen Verbindungen bevorzugt auch inhalativ appliziert. Hierzu werden diese entweder direkt als Pulver (vorzugsweise in mikronisierter Form) oder durch Vernebeln von Lösungen oder Suspensionen, die sie enthalten, verabreicht. Bezüglich der Zubereitungen und Darreichungsformen wird beispielsweise auf die Ausführungen im Europäischen Patent 163 965 verwiesen.

Für die Behandlung von Dermatosen erfolgt die Anwendung der erfindungsgemäßen Verbindungen insbesondere in Form solcher Arzneimittel, die für eine topische Applikation geeignet sind. Für die Herstellung der Arzneimittel werden die erfindungsgemäßen Verbindungen (= Wirkstoffe) vorzugsweise mit geeigneten pharmazeutischen Hilfsstoffen vermischt und zu geeigneten Arzneiformulierungen weiterverarbeitet. Als geeignete Arzneiformulierungen seien beispielsweise Puder, Emulsionen, Suspensionen, Sprays, Öle, Salben, Fettsalben, Cremes, Pasten, Gele oder Lösungen genannt.

Die erfindungsgemäßen Arzneimittel werden nach an sich bekannten Verfahren hergestellt. Die Dosierung der Wirkstoffe bei systemischer Therapie, (p. o. oder i. v) liegt zwischen 0,1 und 10 mg pro Kilogramm und Tag.

## Biologische Untersuchungen

Die dokumentierten pathophysiologischen Effekte der Mastzell-Tryptase werden direkt durch die enzymatische Aktivität der Protease bewirkt. Dementsprechend werden sie durch Inhibitoren, die die enzymatische Aktivität der Tryptose hemmen, reduziert bzw. blockiert. Ein geeignetes Maß für die Affinität eines reversiblen Inhibitors zur Zielprotease ist die Gleichgewichts-Dissoziationskonstante $K_i$ des Enzym-Inhibitor-Komplexes. Dieser $K_i$-Wert kann über den Einfluß des Inhibitors auf die Tryptase-indizierte Spaltung eines chromogenen Peptid-p-Nitroanilid-Substrates oder eines fluorogenen Peptid-Aminomethylcumarin-Substrates bestimmt werden.

## Methodik

Die Dissoziationskonstanten für die Tryptase-Inhibitor-Komplexe werden unter Gleichgewichtsbedingungen entsprechend den allgemeinen Vorschlägen von Bieth (Bieth JG, Pathophysiological Interpretation of kinetic constants of protease inhibitors, Bull. Europ. Physiopath. Resp. 16:183-195, 1980) und den Methoden von Sommerhoff et al. (Sommerhoff CP et al., A Kazaltype inhibitor of human mast cell tryptase: Isolation from the medical leech Hirudo medicinalis, characterization, and sequence analysis, Biol. Chem. Hoppe-Seyler 375: 685-694, 1994) bestimmt.

Menschliche Tryptase wird aus Lungengewebe rein dargestellt; die mittels Titration bestimmte spezifische Aktivität der isolierten Protease beträgt üblicherweise 85 % des theoretischen Wertes. Konstante Mengen der Tryptase werden in Gegenwart von 50 µg/ml Heparin zur Stabilisierung der Protease mit aufsteigenden Mengen der Inhibitoren

inkubiert. Nach Gleichgewichtseinstellung zwischen den Reaktionspartnem wird die verbleibende Enzymaktivität nach Zugabe des Peptid-p-Nitroanilid-Substrates tos-Gly-Pro-Arg-pNA bestimmt, dessen Spaltung Ober 3 min bei 405 nm verfolgt wird. Alternativ kann die enzymatische Restaktivität auch mit fluorogenen Substraten bestimmt werden. Die apparenten Dissoziationskonstanten $K_{Iapp}$ (d.h. in der Gegenwart von Substrat) werden anschließend durch Anpassung der Enzymgeschwindigkeiten an die allgemeine Gleichung für reversible Inhibitoren (Morrison JF, Kinetics of the reversible inhibition of enzymecatalysed reactions by tight-binding inhibitors, Biochim. Biophys. Acta 185, 269-286, 1969) mittels nicht linearer Regression ermittelt:

$$V_I/V_0 = 1 - \{E_t+I_t+K_{Iapp}-[(E_t+I_t+K_{Iapp})^2-4E_tI_t]^{1/2}\}/2E_t$$

Dabei sind $V_I$ und $V_0$ die Geschwindigkeiten in der Gegenwart bzw. Abwesenheit des Inhibitors und $E_t$ und $I_t$ die Konzentrationen der Tryptase und des Inhibitors.

Die für die erfindungsgemäßen Verbindungen ermittelten apparenten Dissoziationskonstanten ergeben sich aus der folgenden Tabelle A, in der die Nummern der Verbindungen den Nummem der Verbindungen in den Beispielen entsprechen.

Tabelle A

| Hemmung der humanen Tryptase | |
|---|---|
| **Verbindung** | **$K_{Iapp}$ (µM)** |
| 1 | 3 |
| 11 | 0,03 |
| 15 | 3 |
| 17 | 22 |
| 21 | 0,1 |
| 23 | 0,8 |
| 31 | 0,2 |
| 34 | 2 |
| 36 | 0,028 |

## Patentansprüche

**1.** Bifunktionelle Inhibitoren von humaner Tryptase der Formel I

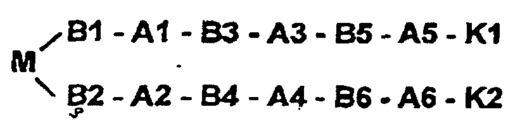

(I)

worin
A1 und A2 gleich oder verschieden sind und -O- (Sauerstoff) oder -NH-C (O)-bedeuten,
A3 und A4 gleich oder verschieden sind und -C(O)-NH- bedeuten oder ausgewählt sind aus der Gruppe

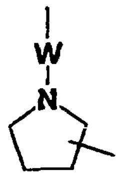 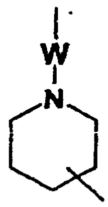 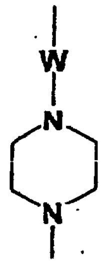

wobei W die Gruppe -C(O)- oder eine Bindung bedeutet,
A5 und A6 gleich oder verschieden sind und -C(O)-, -C(O)-NH-, -NH-C(O)- oder eine Bindung bedeuten,
M ausgewählt ist aus einer der nachfolgenden Gruppen

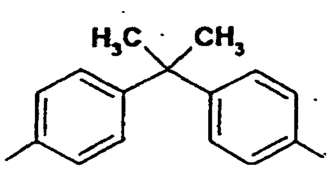 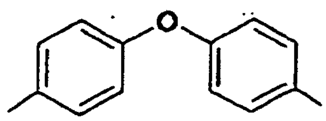 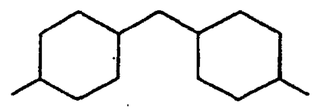

K1 -B7-$(C(O))_m$-B9-X1 oder -B7$(C(O))_m$-B9-Z1-B11-X1 bedeutet,
K2 -B8-$(C(O))_p$-B10-X2 oder -B8$(C(O))_p$-B10-12-B12-X2 bedeutet,
B1, B2, B3, B4, B5 und B6 gleich oder verschieden sind und eine Bindung oder -$CH_2$- (Methylen) bedeuten,
B7, B8, B9, B10, B11 und B12 gleich oder verschieden sind und eine Bindung oder 1-2C-Alkylen bedeuten,
m 0 oder 1 bedeutet,
p 0 oder 1 bedeutet,
X1 und X2 gleich oder verschieden sind und Amino, Amidino oder Guanidino bedeuten,
Z1 und Z2 gleich oder verschieden sind und 1,4-Phenylen, 1,3-Phenylen, 1,4-Cyclohexylen oder 1,4-Piperazinylen bedeuten,
und worin auf direktem Weg zwischen den terminalen Stickstoffatomen 24 bis 40 Bindungen vorhanden sein müssen,
die Salze dieser Verbindungen, wobei alle diejenigen Verbindungen ausgeschlossen sind, bei denen eine oder mehrere der Variablen B1, B2, B3, B4, B5, B6, B7, B8, B9, B10, B11 oder B12 die Bedeutung einer Bindung annehmen und es dadurch zur direkten Verknüpfung zweiter Heteroatome oder zweiter Carbonylgruppen kommen würde.

2. Inhibitoren nach Anspruch 1 mit der chemischen Bezeichnung Bis{4-[4-(4-aminomethylcyclohexanoyl)piperazin-1-yl]carbonyl}-4,4'-diamino-diphenylether, Bis{4-[4-(3-aminomethyl)benzoyl-piperazin-1-yl]carbonyl} 4,4'-diamino-diphenylether, Di{4-[4-(4-aminomethyl)cyclohexanoylamino]piperidin-1-yl-carbamoyl}cyclohexylmethan, 2,2-Bis[4-(4-guanidinylbenzylamino)-carbonylmethoxyphenyl]propan, 2,2-Bis[4-(10-amino-3,6-diaza-2,5-dioxodecyloxy)phenyl]-propan oder 2,2-Bis{4-[4-(4-aminomethylbenzylcarbamoyl)-1-piperazinyl-carbonyloxy]phenyl}-propan, sowie die Salze dieser Verbindungen.

3. Verbindungen der Formel I nach Anspruch 1 zur Behandlung von Krankheiten.

4. Verwendung von Verbindungen der Formel I nach Anspruch 1 zur Herstellung von Medikamenten zur Behandlung von Atemwegserkrankungen.

5. Arzneimittel enthaltend eine oder mehrere Verbindungen der Formel I nach Anspruch 1 zusammen mit üblichen Hilfs- und/oder Trägerstoffen.

## Claims

1. A bifunctional inhibitor of human tryptase of the formula I

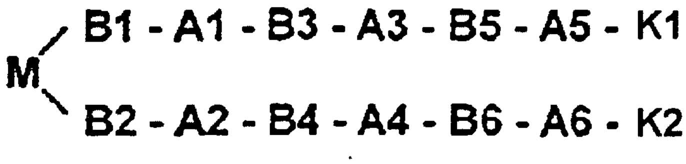

(I)

in which

A1 and A2 are identical or different and are -O-(oxygen) or -NH-C(O)-,
A3 and A4 are identical or different and are -C(O)-NH- or are selected from the group

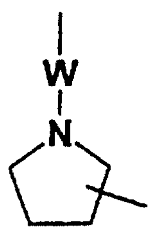 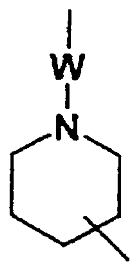 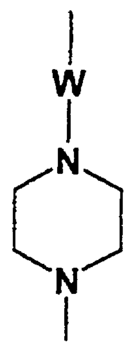

where W is the group -C(O)- or a bond,
A5 and A6 are identical or different and are -C(O)-, -C(O)-NH-, -NH-C(O)- or a bond,
M is selected from one of the following groups

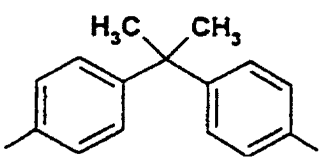 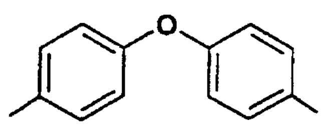 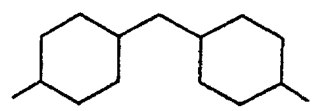

K1 is -B7-$(C(O))_m$-B9-X1 or -B7-$(C(O))_m$-B9-Z1-B11-X1,
K2 is -B8-$(C(O))_p$-B10-X2 or -B8-$(C(O))_p$-B10-Z2-B12-X2,
B1, B2, B3, B4, B5 and B6 are identical or different and are a bond or $-CH_2-$ (methylene),
B7, B8, B9, B10, B11 and B12 are identical or different and are a bond or 1-2C-alkylene,
m is 0 or 1,
p is 0 or 1,
X1 and X2 are identical or different and are amino, amidino or guanidino,
Z1 and Z2 are identical or different and are 1,4-phenylene, 1,3-phenylene, 1,4-cyclohexylene or 1,4-piperazinylene,

and in which 24 to 40 bonds must be present on the direct route between the terminal nitrogen atoms,
the salts of these compounds, with all those compounds being excluded, in which one or more of the variables B1, B2, B3, B4, B5, B6, B7, B8, B9, B10, B11 or B12 assume the meaning of a bond, with this thereby resulting in the direct linkage of two heteroatoms or two carbonyl groups.

**2.** An inhibitor as claimed in claim 1 having the chemical designation bis{4-[4-(4-aminomethylcyclohexanoyl)piperazin-1-yl]carbonyl}-4,4'-diaminodiphenyl ether, bis{4-[4-(3-aminomethyl)benzoylpiperazin-1-yl]-carbonyl}-4,4'-diaminodiphenyl ether, di{4-[4-(4-aminomethyl)cyclohexanoylamino]piperidin-1-ylcarbamoyl}cyclohexylmethane, 2,2-bis[4-(4-guanidinylbenzylamino)carbonylmethoxyphenyl]propane, 2,2-bis[4-(10-amino-3,6-diaza-2,5-dioxodecyloxy)phenyl]propane or 2;2-bis{4-[4-(4-aminomethylbenzylcarbamoyl)-1-piperazinylcarbonyloxy]phenyl}propane, and also the salts of these compounds.

3. A compound of the formula I as claimed in claim 1 for the treatment of diseases.

4. The use of a compound of the formula I as claimed in claim 1 for the production of medicaments for the treatment of respiratory disorders.

5. A medicament comprising one or more compounds of the formula I as claimed in claim 1 together with customary auxiliary and/or carrier substances.

## Revendications

1. Inhibiteurs bifonctionnels de tryptase humaine de formule I

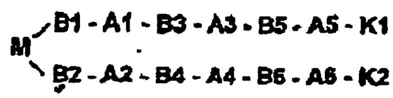

(I)

dans laquelle
A1 et A2 sont identiques ou différents et représentent -O - (oxygène) ou -NH-C(O)-.
A3 et A4 sont identiques ou différents et représentent -C(O)-NH- ou sont choisis dans le groupe

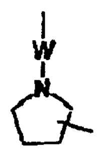 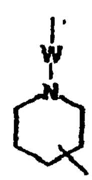 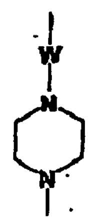

où W représente le groupe -C(O)- ou une liaison,
A5 et A6 sont identiques ou différents et représentent -C(O)-, -C(O)-NH-, -NH-C(O)- ou une liaison,
M est choisi dans l'un des groupes suivants

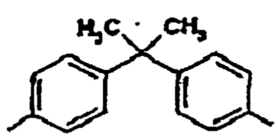 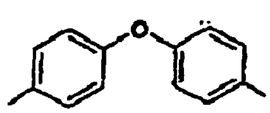 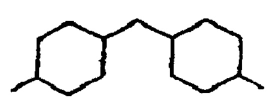

K1 représente -B7-$(C(O))_m$-B9-X1 ou -B7$(C(O))_m$-B9-Z1-B11-X1,
K2 représente -B8-$(C(O))_p$-B10-X2 ou -B8$(C(O))_p$-B10-Z2-B12-X2,
B1, B2, B3, B4, B5 et B6 sont identiques ou différents et représentent une liaison ou -$CH_2$- (méthylène),
B7, B8, B9, B10, B11 et B12 sont identiques ou différents et représentent une liaison ou le 1-2-C- alkylène,
m représente 0 ou 1,
p représente 0 ou 1
X1 et X2 sont identiques ou différents et signifient-amino, amidino ou guanidino,
Z1 et Z2 sont identiques ou différents et signifient 1,4-phénylène, 1,3-phénylène, 1,4-cyclohexylène ou 1,4-pipérazinylène,
et dans laquelle de 24 à 40 liaisons doivent être présentes sur le chemin direct entre les atomes d'azote terminaux, les sels de ces composés, à l'exclusion de tous les composés dans lesquels une ou plusieurs des variables B1, B2, B3, B4, B5, B6, B7, B8, B9, B10, B11 ou B12 adoptent la signification d'une liaison, ce qui entraînerait la liaison directe de deux hétéroatomes ou de deux groupes carbonyles.

2. Inhibiteurs selon la revendication 1 ayant pour nom chimique bis{4-[4-(4-aminométhylcyclohexanoyl)pipérazin-1-yl]carbonyl}-4,4'-diamino-diphényléther, bis{4-[4-(3-aminométhyl)benzoyl-pipérazin-1-yl]carbonyl}-4,4'-diamino diphényléther, di {4-[4-(4-aminométhyl)cyclohexanoyl-amino]pipéridin-1-yl-carbamoyl}cyclohexylméthane, 2,2-bis[4-(4-guanidinylbenzylamino)-carbonylméthoxyphényl]-propane, 2,2-bis[4-(10-amino-3,6-diaza-2,5-dioxodécyloxy)phényl]-propane ou 2,2-bis{4-[4-(4-aminométhylbenzylcarbamoyl)-1-pipérazinyl-carbonyloxy]-phényl} propane, ainsi que les sels de ces composés.

**3.** Composés de formule I selon la revendication 1 pour le traitement de maladies.

**4.** Utilisation de composés de formule I selon la revendication 1 pour fabriquer des médicaments pour le traitement des maladies des voies respiratoires.

**5.** Médicament contenant un ou plusieurs des composés de formule I selon la revendication 1 ensemble avec les adjuvants et/ou excipients usuels.

**Pyridin-2,6-dicarbonsäure-bis-[4-(aminomethyl-benzoyl)-1-piperazid] (1)**

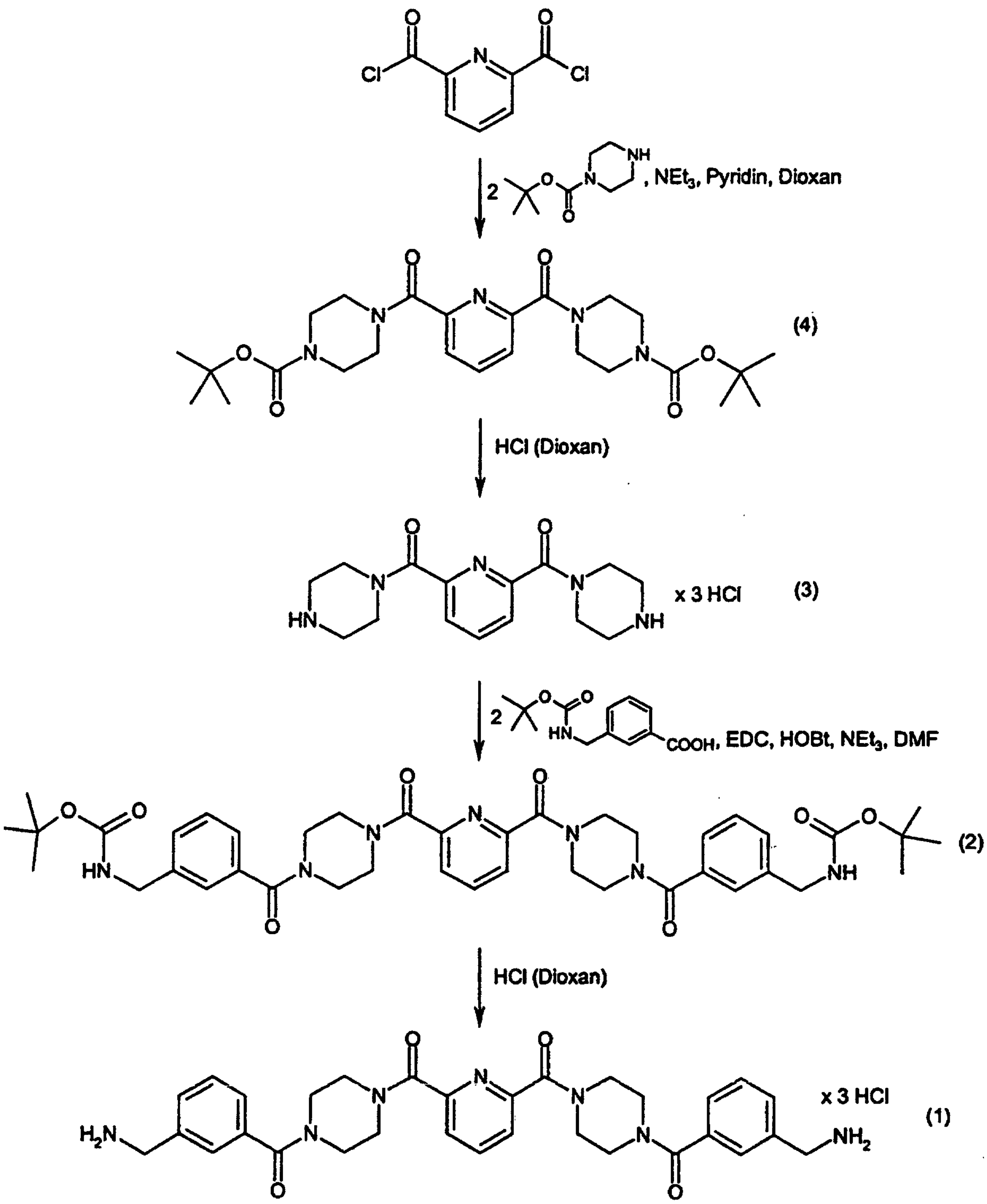


**Figur 1**

**Pyridin-2,6-dicarbonsäure-bis-[4-(trans-4-aminomethyl-cyclohexylcarbonyl)-1-piperazid] (5)**

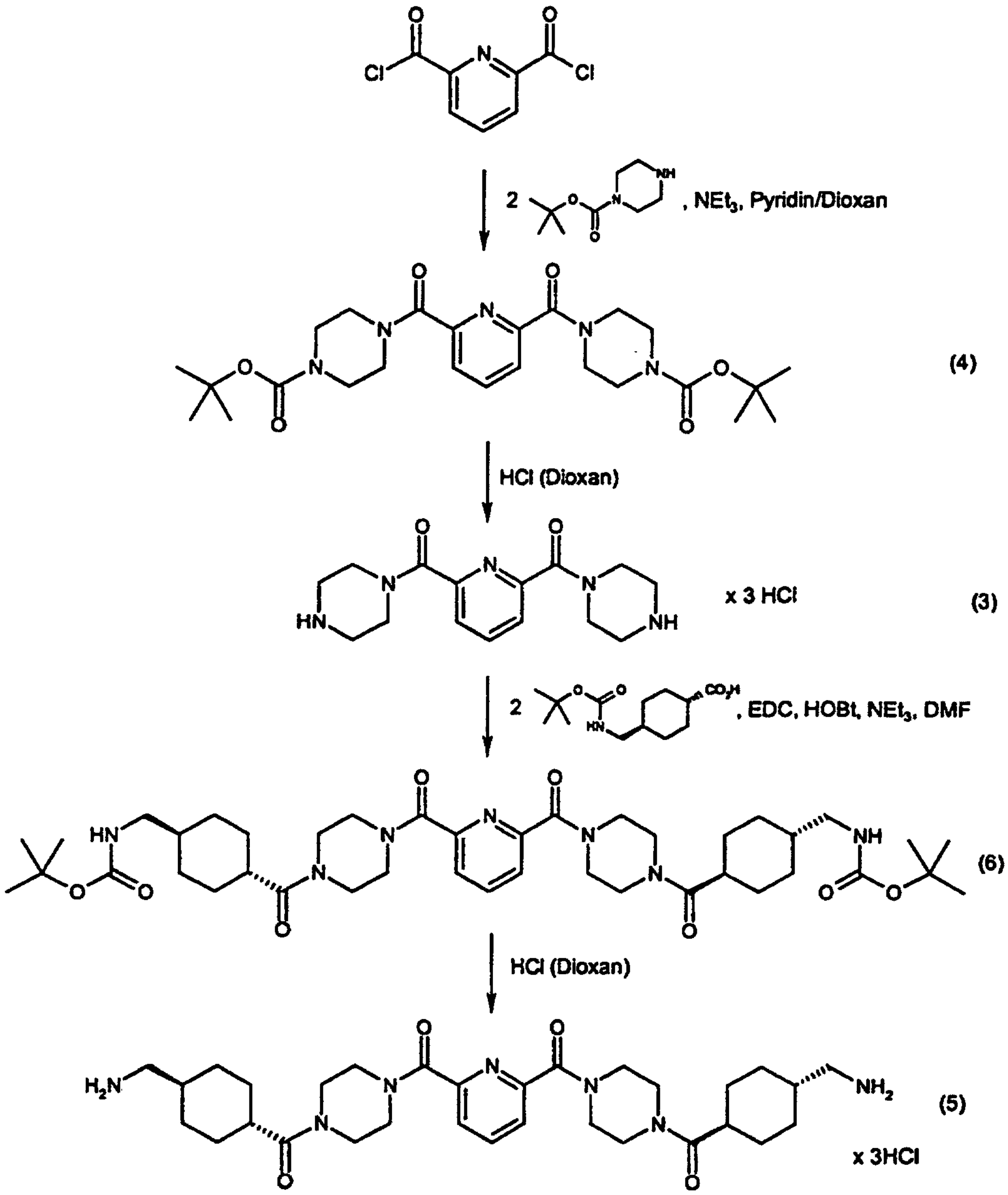


(4)

(3)

(6)

(5)

**Figur 2**

**2,6-Dimethyl-4-phenyl-pyridin-3,5-dicarbonsäure-bis-[4-(3-aminomethyl-benzoyl)-1-piperazid] (7)**

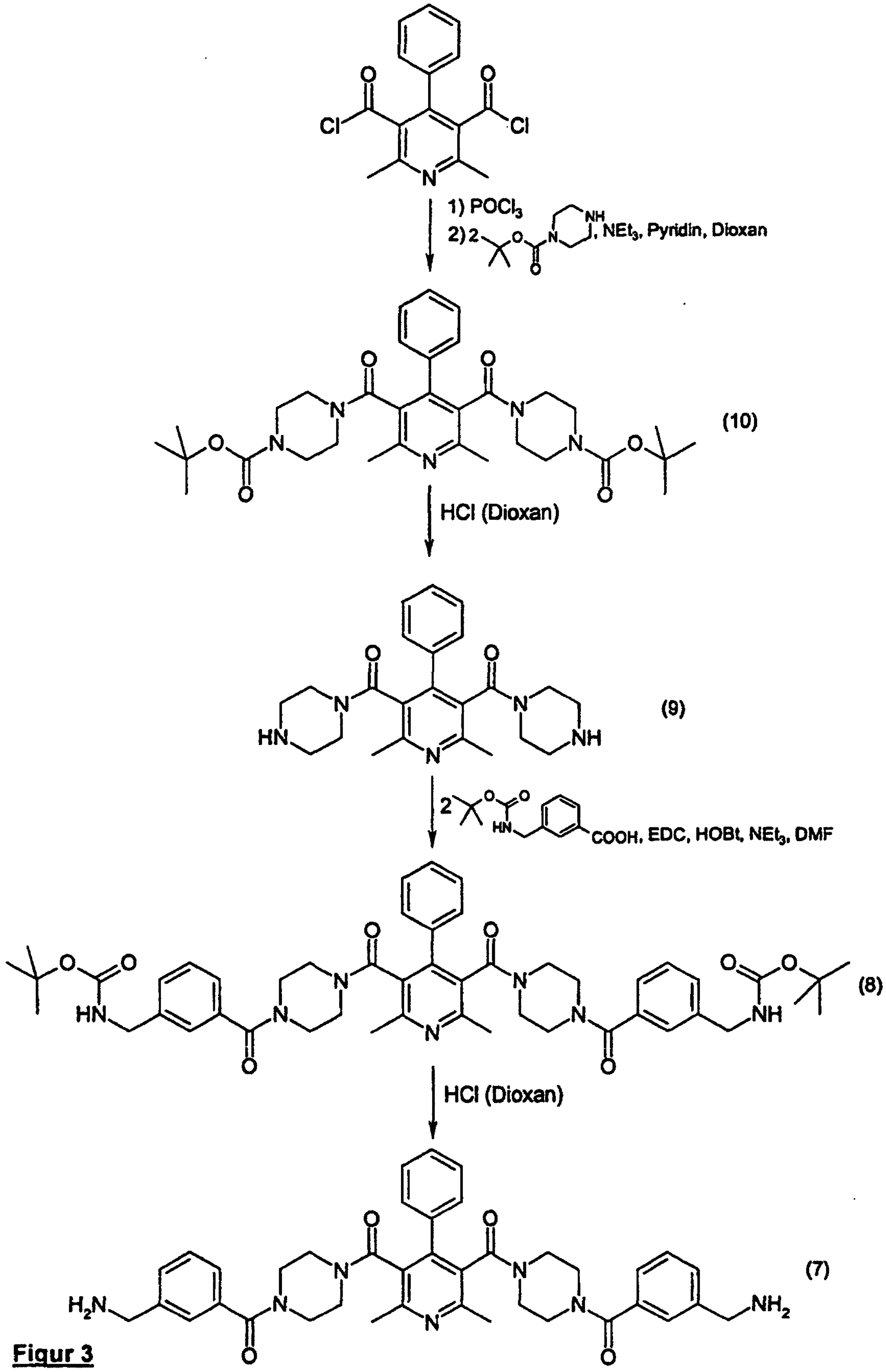


(10)

(9)

(8)

(7)

**Figur 3**

**Pyridin-2,6-dicarbonsäure-bis-[4-(3-aminomethyl-benzoylamino)-1-piperidid] (11)**

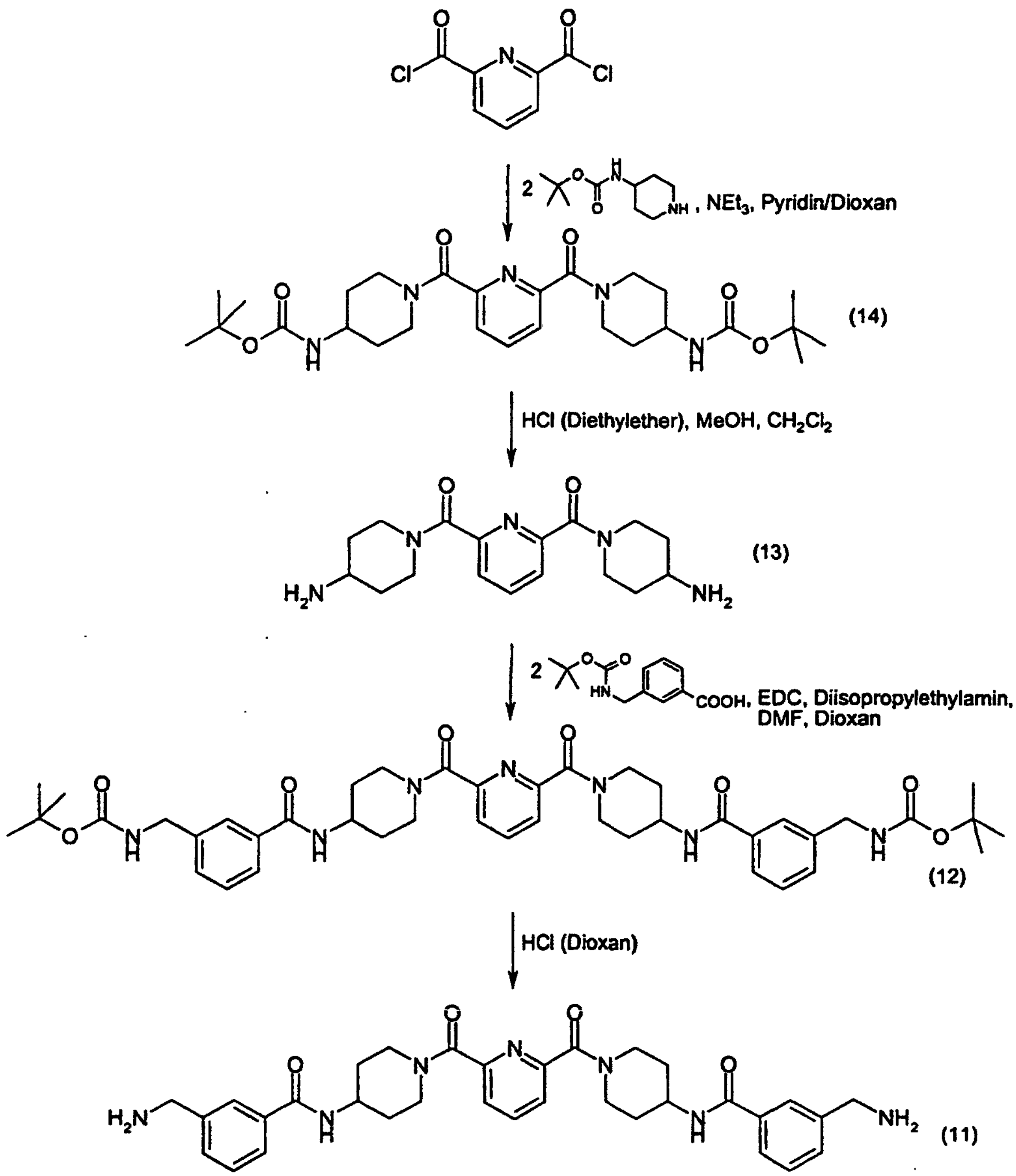


(14)

(13)

(12)

(11)

**Figur 4**

**Pyridin-2,6-dicarbonsäure-bis-[4-(4-aminomethyl-cyclohexylcarbonylamino)-1-piperidid] (15)**

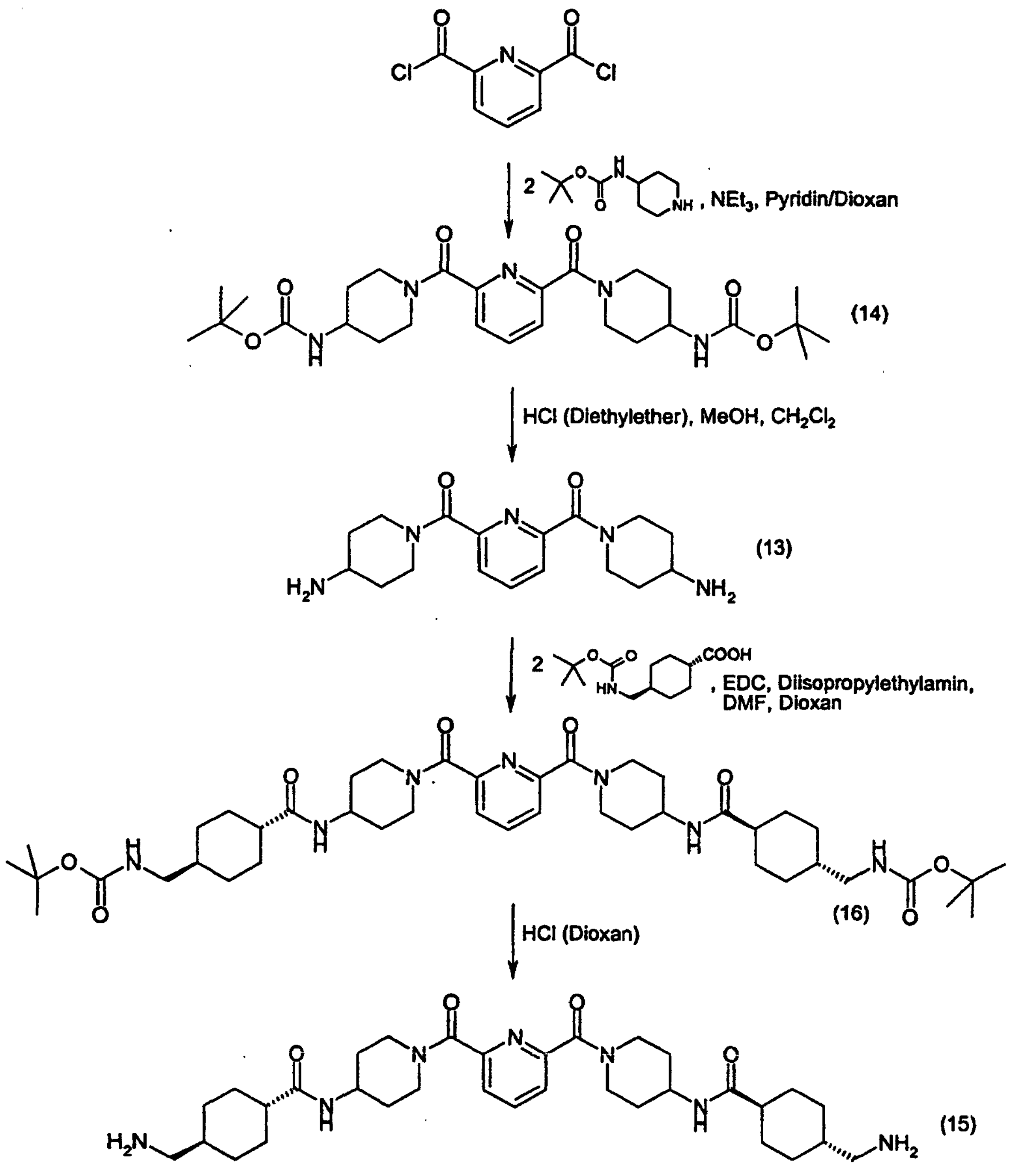


(14)

(13)

(16)

(15)

**Figur 5**

**Bis{4-[4-(4-aminomethylcyclohexanoyl)piperazin-1-yl]carbonyl}4,4'-diamino-diphenylether Dihydrochlorid (17)**

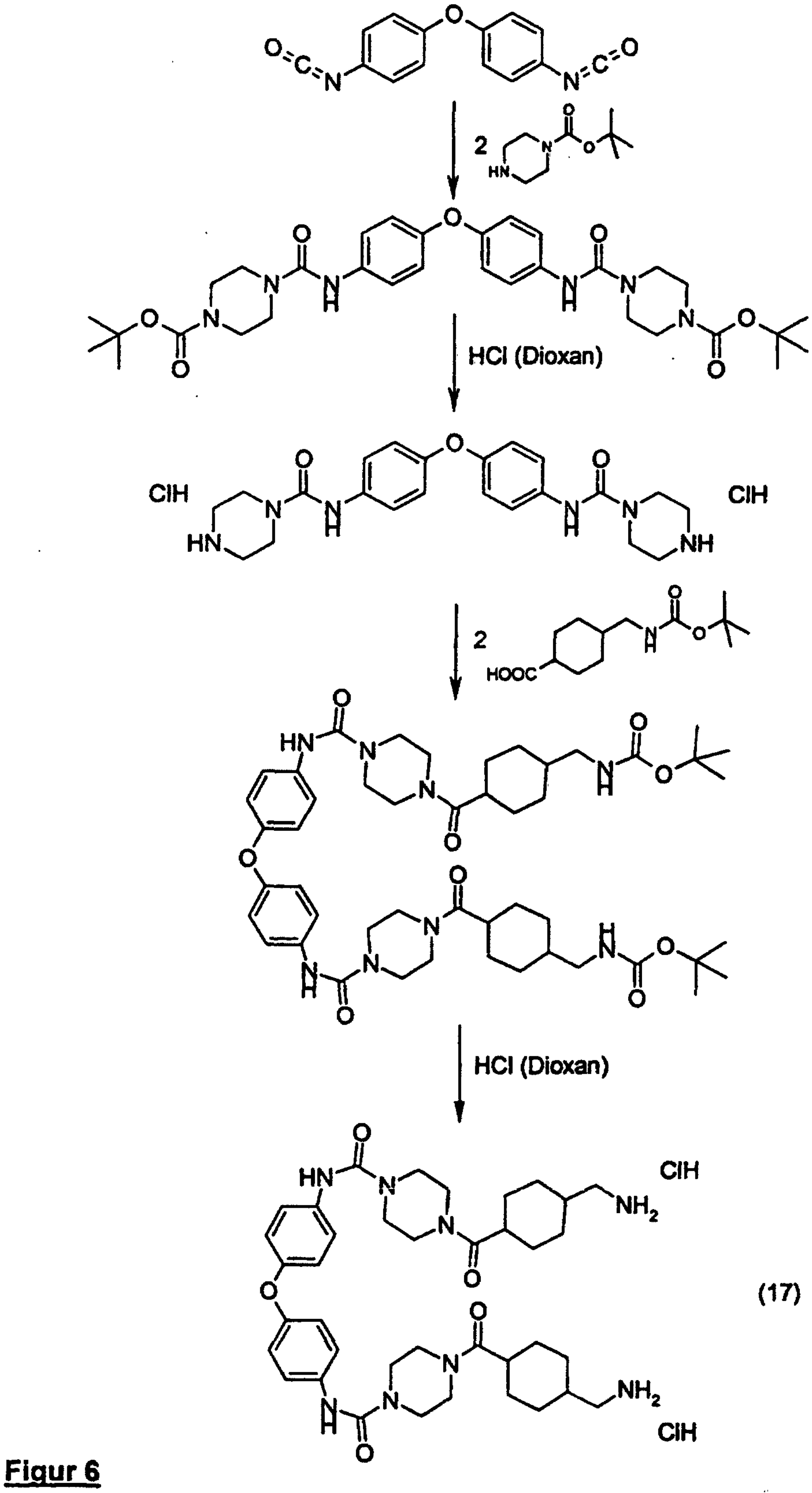


(20)

(19)

(18)

(17)

**Figur 6**

**Bis{4-[4-(3-aminomethyl)benzoyl-piperazin-1-yl]carbonyl}4,4'-diamino-diphenylether Dihydrochlorid (21)**

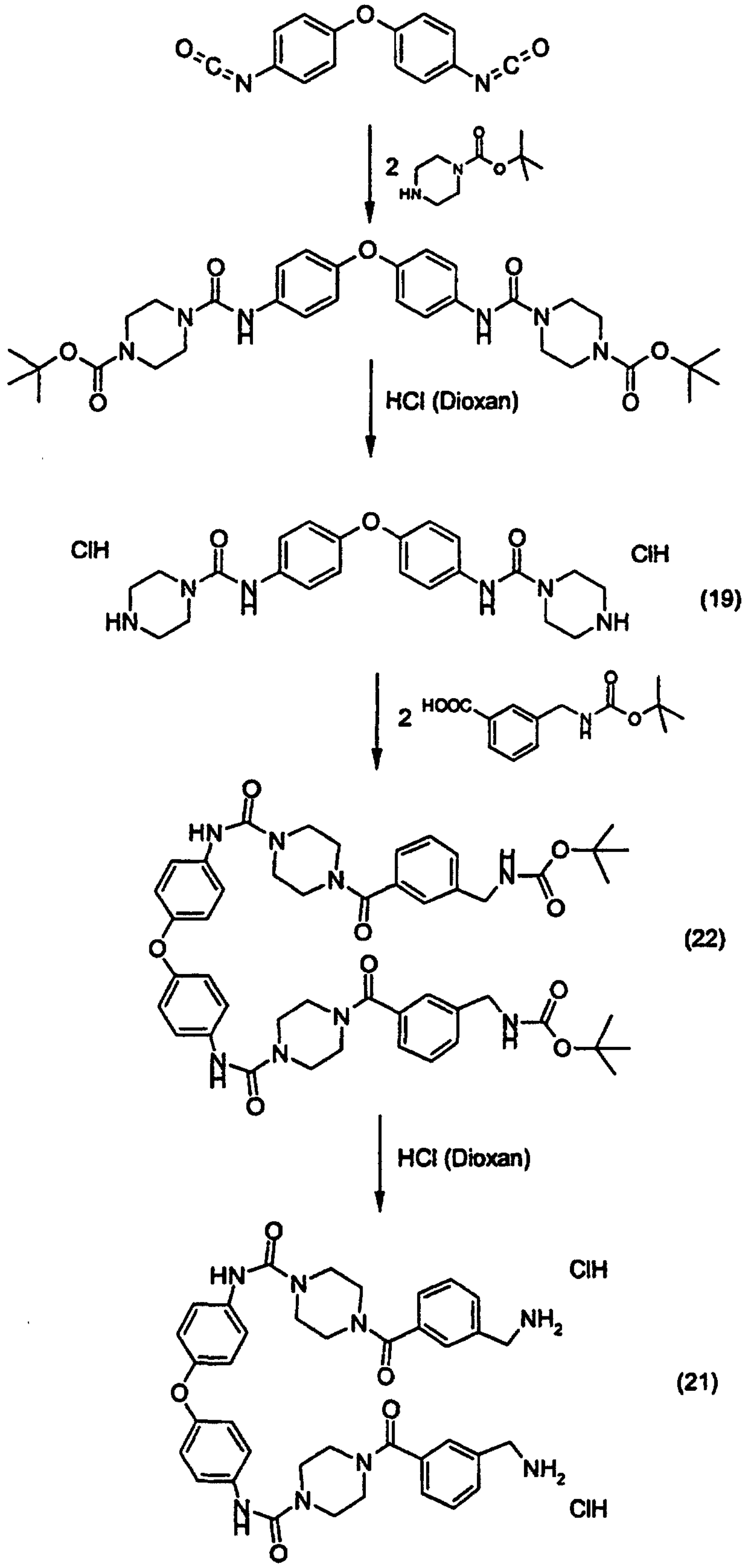


(20)

(19)

(22)

(21)

**Figur 7**

Di{4-[4-(4-aminomethyl)cyclohexanoylamino]piperidin-1-yl-carbomoyl}-cyclohexylmethan Dihydrochlorid (23)

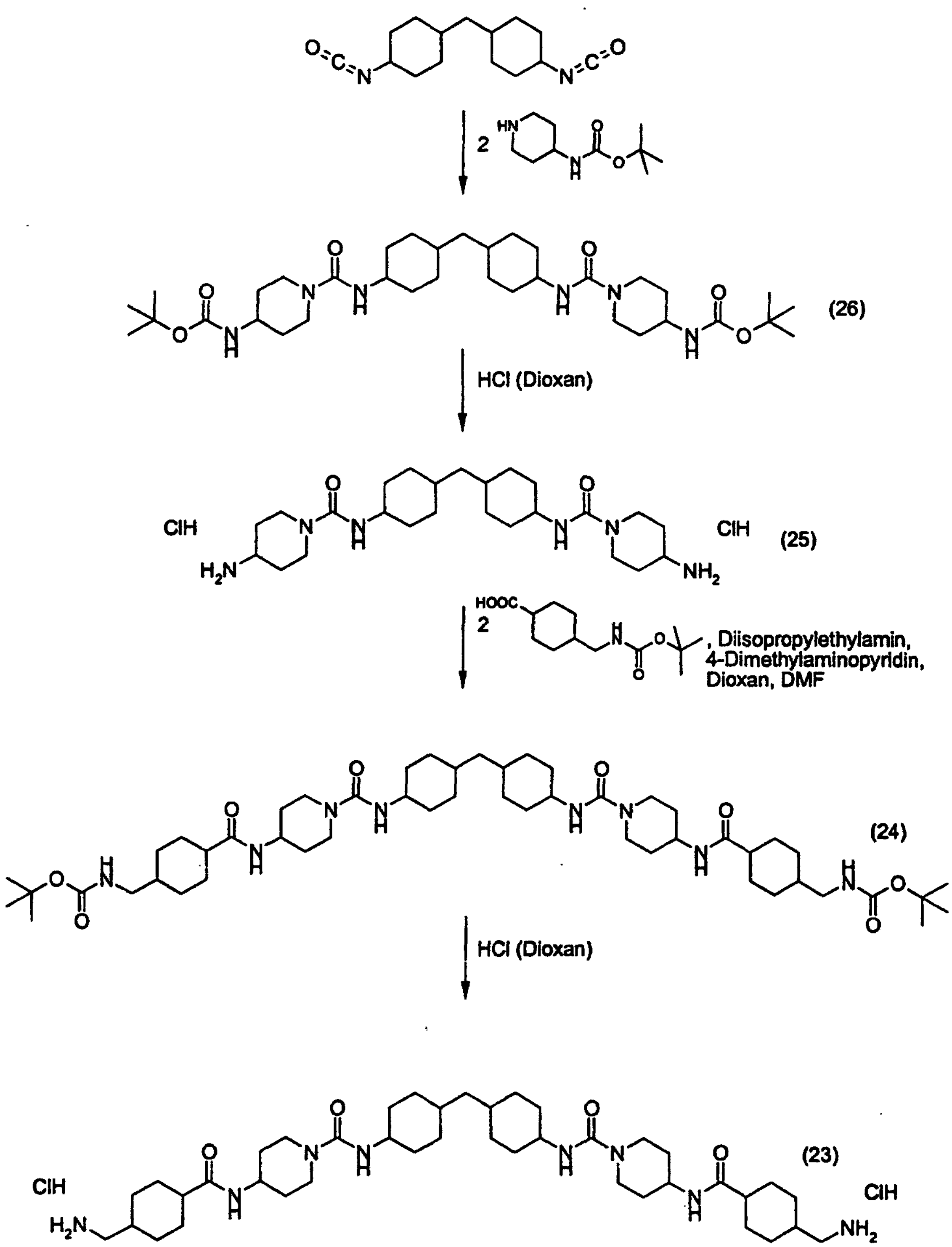


(26)

(25)

(24)

(23)

**Figur 8**

**2,2-Bis{4-[4-(4-aminophenyl)-1-piperazinylcarbonyl-methoxy]phenyl}propan Dihydrochlorid (27)**

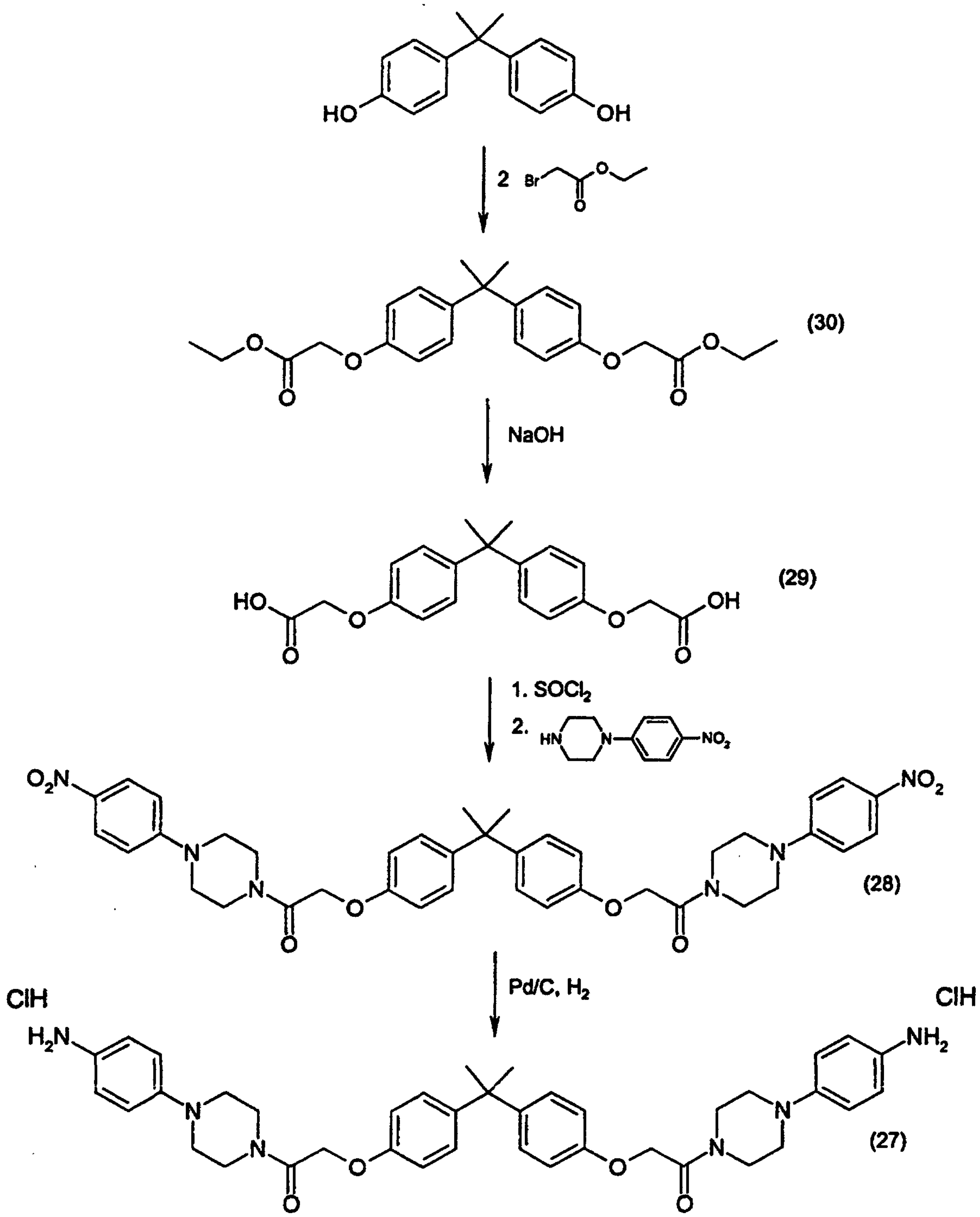


(30)

(29)

(28)

(27)

**Figur 9**

**2,2-Bis-[4-(4-guanidyl-benzylamino)carbonylmethoxyphenyl]propan-dihydroacetat (31)**

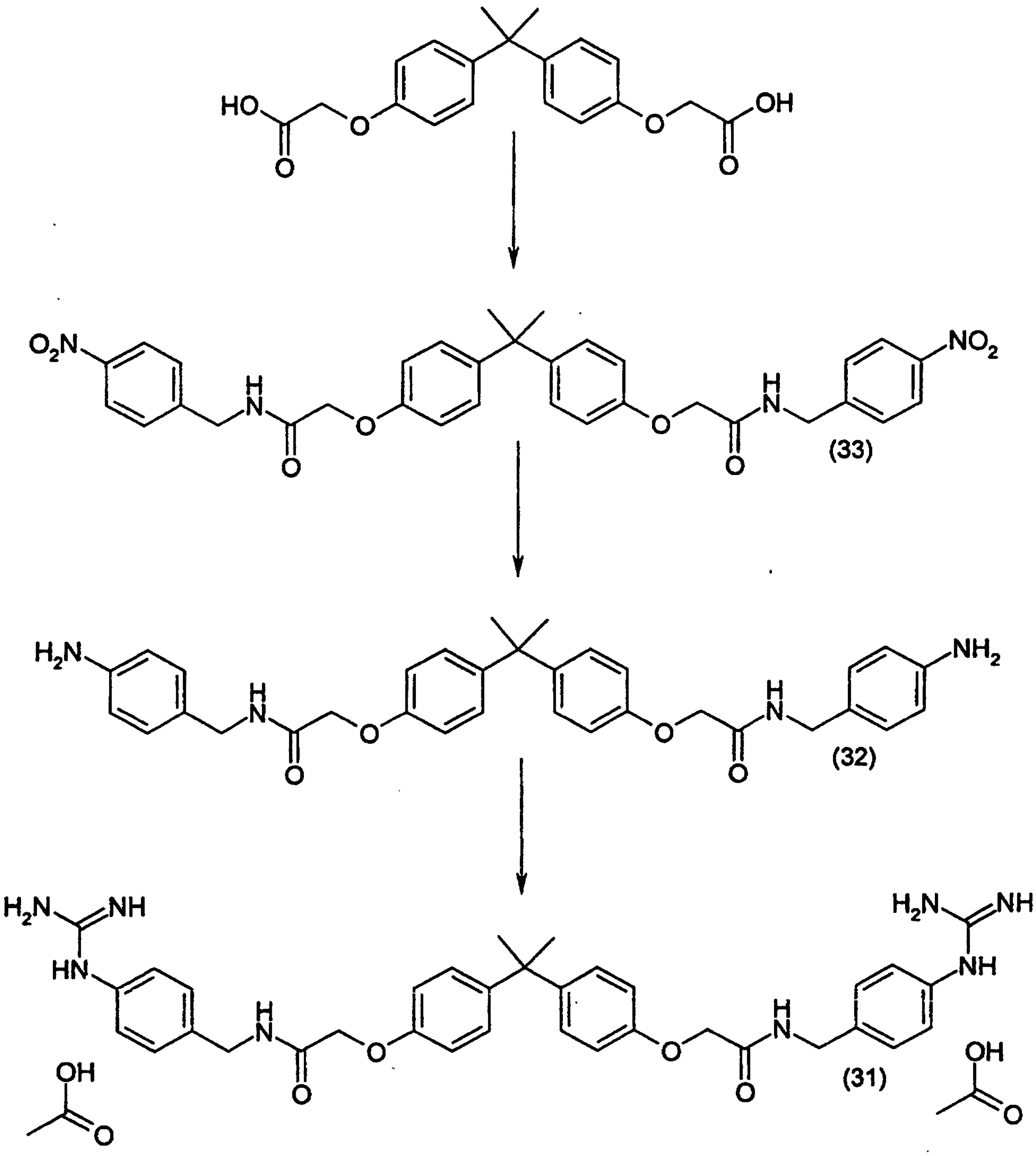

(33)

(32)

(31)

**Figur 10**

**2,2-Bis-[4-(10-amino-3,6-diaza-2,5-dioxodecyloxy)phenyl]propan-dihydrochlorid (34)**

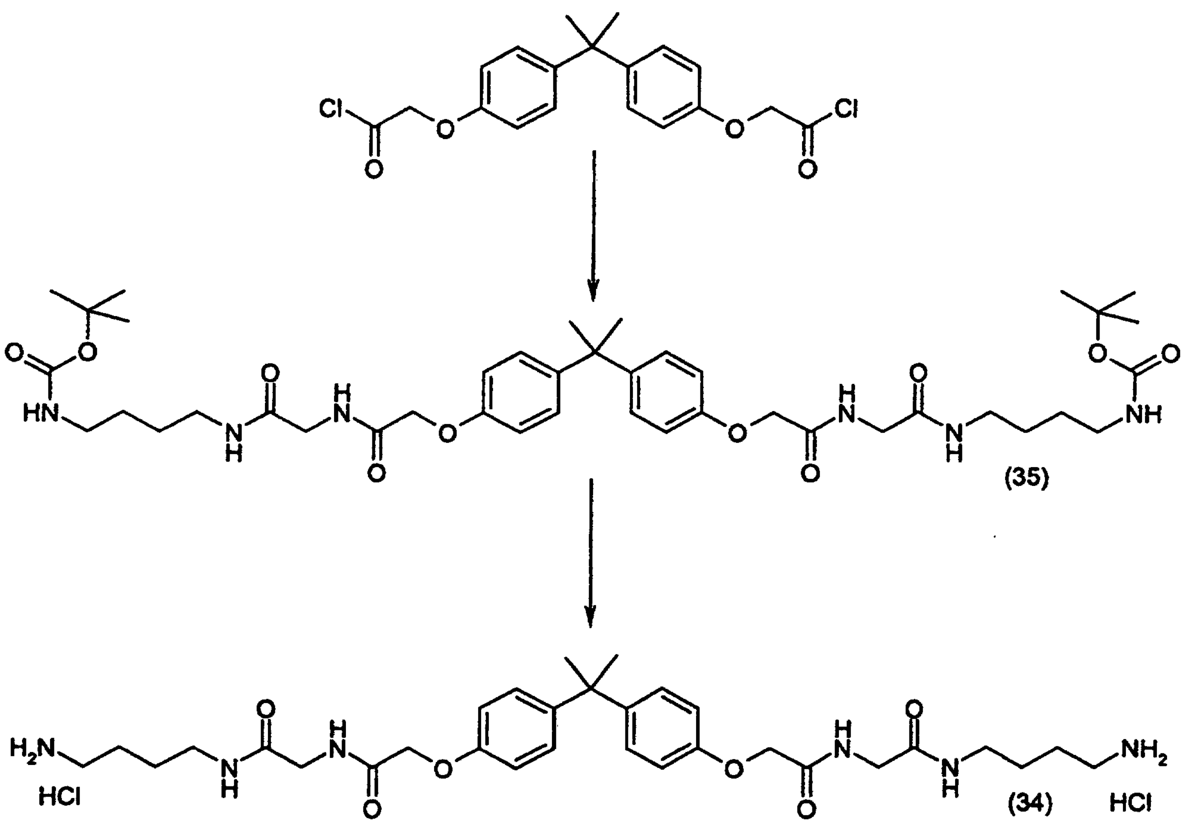

(35)

(34)

**Figur 11**

**2,2-Bis-{4-[4-(4-aminomethylbenzylcarbamoyl)-1-piperazinylcarbonyloxy]phenyl}propan (36)**

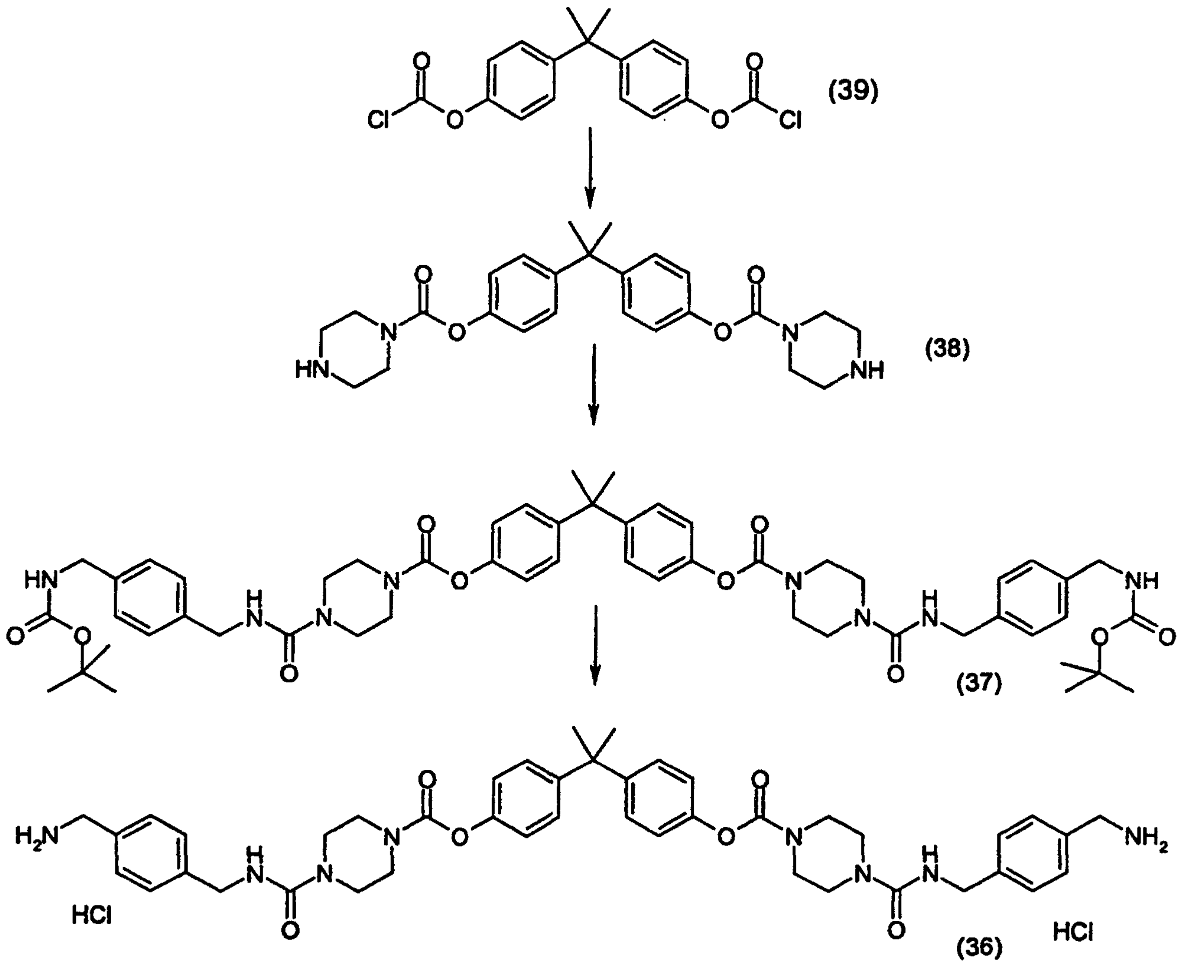

(39)

(38)

(37)

(36)

**Figur 12**